# EUROPEAN PATENT SPECIFICATION

(11) **EP 3 766 455 B1**
(45) Date of publication and mention of the grant of the patent: **11.11.2026**
(21) Application number: 20195203.3
(22) Date of filing: 12.06.2013
(51) Int. Cl.: A61D 1/02, A61D 7/00, A61F 9/007, A61M 5/31, A61M 5/315

(54) **INTRAOCULAR GAS INJECTOR**
INTRAOKULARER GASINJEKTOR
INJECTEUR DE GAZ INTRAOCULAIRE

(30) Priority: 12.06.2012 US 201261658765 P; 15.03.2013 US 201361799840 P
(43) Date of publication of application: 20.01.2021
(62) Document number(s) of the earlier application(s) in accordance with Art. 76 EPC: 13803578.7 / 2 858 593
(73) Proprietor: Alcon Inc., 1701 Fribourg (CH)
(72) Inventor: AULD, Jack R., Laguna Niguel, CA 92677 (US); HUCULAK, John C., Mission Viejo, CA 92692 (US); McCOLLAM, Christopher L, Fountain Valley, CA 92708 (US)
(74) Representative: Murgitroyd & Company

(56) References cited:
- WO-A1-2004/037335
- WO-A1-2011/011423
- US-A- 5 256 154

## Description

### RELATED APPLICATIONS

The present application claims priority to U.S. Provisional Application No. 61/658,756 filed June 12, 2012, entitled INTRAOCULAR GAS INJECTOR and U.S. Provisional Application No. 61/799,840 filed March 15, 2013, entitled INTRAOCULAR GAS INJECTOR.

### TECHNICAL FIELD

The inventions disclosed herein generally relate to devices for injecting gases into an eye of an animal.

### BACKGROUND OF THE INVENTIONS

Surgical procedures can require gases or other fluids to be injected into a target area for treatment of certain injuries, disorders and diseases. In the treatment of eye conditions such as macular holes, retinal tears and detachments, part of the surgical procedure can include the injection of gases or other fluids into the eye.

For example, retinal detachment is an eye disorder involving the separation of the retina from the Retinal Pigment Epithelium (RPE), the tissue that holds the retina in place. Retinal detachment can occur due to a retinal tear, traction on the retina, or inflammation which allows fluid to build up in the subretinal space thereby causing the retina to begin to separate from supporting RPE tissue. This disorder can also occur due to Posterior Vitreous Detachment (PVD), Proliferative Diabetic Retinopathy (PDR), injury, or neovascularization of the fibrous or vascular tissue causing the retina to be detached from the RPE. Such a condition, if not treated immediately, could lead to partial vision loss and potentially even blindness.

Treatment approaches for uncomplicated retinal detachments may include non-surgical techniques such as pneumatic retinopexy, laser photocoagulation, or cryopexy. More complicated retinal detachments require surgical intervention. Due to the risk of infection, which can potentially cause blindness, such surgeries are performed under sterile conditions in order to significantly reduce the potential for infection. Surgical methods include vitrectomy, which is the removal of the vitreous humor; dissection and removal of membranes, in the case of traction retinal detachments; and photocoagulation or cryopexy, in the case of additional retinal tears. Following such a surgical procedure, an intraocular gas tamponade may be used to hold the retina tissue in contact with the RPE which enables the retina to remain attached during the healing process after the surgical procedure.

Since intraocular pressure must be maintained relatively constant during the healing process, the gas chosen is typically one that expands at constant pressure (isobaric process). As such, the intraocular gas tamponade can be a gas bubble of air mixed with an expansile gas such as sulfur hexafluoride (SF₆), hexafluroethane (C₂F₆), or octafluoropropane (C₃F₈). The intraocular gas tamponade dissolves over time depending on the gas and concentrations used. For example, sulfur hexafluoride dissolves within 1-2 weeks when mixed with air at a concentration of approximately 20 percent, hexafluoroethane dissolves in approximately 4-5 weeks when mixed with air at a concentration of approximately 16 percent, and octafluoropropane dissolves in approximately 6-8 weeks when mixed with air at a concentration of approximately 12%. Changing the concentrations of these gases affects the duration.

Current practice involves use of gases contained in separate, multi-dose pressurized containers which are then transferred into a syringe for mixing with air and injection into the patient's eye. Therefore, during a surgical procedure, multiple non-sterile and sterile steps are required in order to fill the syringe with a desired concentration of gas and air. These non-sterile and sterile steps are typically performed by the non-sterile operating room circulating nurse and the sterile scrub technician supporting the surgeon in the sterile field. During a first non-sterile step, the circulating nurse prepares the non-sterile re-usable gas container by setting a pressure regulator connected to the gas container at the proper pressure. During a second step, the scrub tech prepares a sterile syringe by connecting a stopcock, filter, and tubing, in series, onto the syringe. During a third step, the tubing is connected to the gas container. The scrub tech carefully passes the free end of the sterile tubing through the invisible sterile barrier to the awaiting non-sterile circulating nurse. The non-sterile circulating nurse receives the tubing and carefully ensures that he/she does not contaminate the scrub tech nor any other of the sterile surfaces; and connects the tubing to the regulator on the gas container. During a fourth step, the syringe is then filled with gas from the container. The scrub tech and circulating nurse coordinate the opening of the pressurized container valve to release gas through the connected tubing, filter, stopcock, and into the syringe. The pressure of the released gas is sufficient to push the syringe plunger along the length of the syringe barrel and thus fill the syringe with gas. The scrub tech ensures that the gas does not push the plunger out of the open end of the syringe barrel and signals to the circulating nurse to close the gas container valve when the syringe approaches a fully filled condition. During a fifth step, the syringe is then purged of all air and gas in order to ensure that a substantial majority of air which may have been present within the syringe, stopcock, filter, and tubing, prior to filling with gas has been purged. The scrub tech turns the stopcock, to provide a means for the air and gas in the syringe to be released to the atmosphere, presses on the syringe plunger, and empties the syringe of all of its contents. The scrub tech then turns the stopcock in the opposite direction, returning the connection pathway to the tubing and the gas container. Steps four and five are repeated several times to further reduce the amount of air that was initially in the syringe, stopcock, filter, and tubing; flushing the majority of the air from the syringe, stopcock, filter, and tubing and purging the system of air. During a sixth step, the syringe is then refilled with gas from the container. The scrub tech detaches the tubing from the filter and signals the circulating nurse to carefully take the tubing, removing it from the sterile field. During a seventh step, the scrub tech does not expel the full contents of the syringe, stopping the plunger such that only a measured volume of gas remains in the syringe. For example, the gas may be expelled such that only 12mL remains within the syringe. During an eighth step, the scrub tech replaces the used filter with a new sterile filter and draws filtered room air into the syringe until the total air/gas mixture in the syringe is at a proper volume for the desired gas concentration.

For example, atmospheric air may be drawn into the syringe such that the total volume of air and gas is 60mL therefore achieving a concentration of 20 percent. Since the pressurized containers are non-sterile, and the syringe and surgical area are sterile, completing the above-mentioned steps must be performed by at least one party in the non-sterile field (typically the circulating nurse), a second party in the sterile field (typically the scrub tech), and requires the coordination and communication between the two parties.

The procedure requires a complex set of steps which may increase the potential for errors occurring. An error in one of these steps can result in an improper concentration of gas being used which may result in having either an elevated pressure or reduced retinal tamponade duration thereby potentially causing ischemia or failure of the reattachment surgery, both of which potentially causing blindness. Additionally, the current practice results in a significant amount of wasted gas which is both expensive and harmful to the environment. Thus handling of such gases, especially in pressurized containers containing more than one dose, may present potential danger to the operator if mishandled. As such, some countries may even prohibit storage of these pressurized containers in the operating room.

While there have been some approaches to improve the current procedure, such as U.S. Patent Number 6,866,142 to Lamborne et al., single-dose containers capable of being placed in the sterile field, and the Alcon^{®} Constellation^{®} system which allows filling and purging of gas, these approaches have been insufficient to address all the potential issues. As such, there remains a need in the industry for an improved gas mixing apparatus.

Reference is made to the cited prior art document US 5 256 154 A, which discloses a pre-filled plastic syringe containing a liquid product for terminal sterilization comprising: a syringe barrel equipped with a one-way valve and a slidable plunger to equalize pressure differentials.

### SUMMARY OF THE INVENTION

An aspect of at least one of the inventions disclosed herein includes the realization that an intraocular gas injector design can allow a surgeon or a nurse to prepare a gas mixture with a selected concentration level using with a simplified procedure. For example, in some known intraocular gas injector devices and procedures, such as conventional syringes, multiple parties can be required to fill the syringe to achieve a desired concentration with one person repeatedly filling and discharging the syringe and another person controlling the flow of a gas contained in an external tank. Additionally, each person must coordinate their actions and perform a multitude of complex steps. This increases the potential for errors in the filling process which could result in an improper concentration being achieved in the syringe prior to injection into a patient. Furthermore, this can increase the time necessary to fill the syringe as the two parties must coordinate their activities and perform multiple steps. The potential for error can be particularly dangerous in certain medical fields, such as ophthalmology, where injection of an improper concentration can result in blindness. Thus, an intraocular gas injector that can be operated by a single person can help reduce the likelihood of error.

Another aspect of at least one of the inventions disclosed herein includes the realization that an intraocular gas injector design can allow for multiple selectable concentration levels thereby allowing one device to be used for different applications and thus potentially further reducing manufacturing costs and waste. For example, some conventional devices can only allow for a preset concentration level to be achieved within the device thereby necessitating the manufacturing and storage of multiple devices having different preset concentration level. This increases both the costs of manufacturing and the cost to a surgeon who needs to purchase multiples of each device to accommodate for different surgical needs. Under such circumstances, some devices can expire thereby requiring the manufacturer or surgeon to dispose of such devices without ever having been used. As such, an intraocular gas injector that allows for multiple concentration levels can serve as a one-size fits all for a surgeon thereby reducing waste.

Another aspect of at least one of the inventions disclosed herein includes the realization that an intraocular gas injector design can allow for automated operation during at least some phases of operation thereby reducing the potential for errors in achieving a proper concentration level. For example, in some known intraocular gas injector devices and procedures, such as conventional syringes, a nurse or other operating room personnel must physically measure the amount of gas contained within a syringe during a first phase of operation. In a situation where a minute change in volume can result in a significant change in concentration, a minor error in this physical measurement can result in an improper concentration being achieved in the syringe after all phases of operation have been completed. Therefore, an intraocular gas injector that automatically measures the volume in the first phase and/or any other phase can reduce the likelihood of an improper concentration being achieved in the injector.

Another aspect of at least one of the inventions disclosed herein includes the realization that an intraocular gas injector design can be made with a canister within the injector, with the canister capable of being filled separately prior to incorporation into the injector, thereby reducing the costs of manufacture. Thus, for example, an intraocular gas injector device can include a separate canister placed within the body of the injector.

Yet another aspect of at least one of the inventions disclosed herein includes the realization that an intraocular gas injector design can incorporate a storage member having an internal valve mechanism thereby reducing the costs of manufacture of the injector. For example, in some designs, the device can use multiple pressure regulation systems, such as check valves, integrated on components of the device to regulate pressure within the device and to control the operation of the device. The integration of pressure regulation systems on components of the device can result in increased manufacturing costs for those components. Thus, the relocation of valves to the storage member can help reduce manufacturing costs of the device if the cost of manufacturing of an internal valve mechanism within the storage member is lower than the cost of manufacturing a valve integrated on other components of the device.

An aspect of at least one of the inventions disclosed herein includes the realization that an intraocular gas injector design can incorporate an interlock mechanism configured to control the movement of an activation switch thereby reducing potential erroneous operation of the injector. For example, in some designs, the device can include an activation switch which can control the operation of the device such as the opening and closing of a pressurized chamber within the device. In some instances, the user can use the activator switch to reopen the pressurized chamber within the device when such opening can cause an improper concentration to be received. An interlock mechanism configured to control the movement of an activation switch can help reduce the likelihood of erroneous operation of the injector.

An aspect of at least one of the inventions disclosed herein includes the realization that an intraocular gas injector design can allow for automated operation during at least some phases of operation when connected to an external pressurized chamber thereby reducing the potential for errors in achieving a proper concentration level. For example, in some known intraocular gas injector devices and procedures, such as conventional syringes, a nurse or other operating room personnel can connect an external gas tank to the syringe and repeatedly fill and discharge gas within the syringe to ensure that the syringe contains mainly gas from the tank. During these refill and discharge cycles, another party may need to open and close a valve on the external gas tank. Thus, for example, an intraocular gas injector can allow the attachment of an external pressurized chamber and automatically reach a configured volume and discharge gas such that the injector contains mainly gas from the external pressurized chamber.

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

### BRIEF DESCRIPTION OF THE DRAWINGS

FIGURE 1 is a first embodiment of a gas mixture apparatus.
FIGURE 2A is a second embodiment of a gas mixture apparatus shown in an initial phase of operation.
FIGURE 2B is a second embodiment of a gas mixture apparatus shown in a first phase of operation.
FIGURE 2C is a second embodiment of a gas mixture apparatus shown in a second phase of operation.
FIGURE 2D is a second embodiment of a gas mixture apparatus shown in a third phase of operation.
FIGURE 3 is an exploded view of the components of the second embodiment of a gas mixture apparatus.
FIGURE 4 is a perspective view of a measurement control system and activation system of the second embodiment of a gas mixture apparatus.
FIGURE 5A is a perspective view of a metering dial of the second embodiment of a gas mixture apparatus.
FIGURE 5B is a sectional view of a metering dial of the measurement control system of Figure 4.
FIGURE 6 is a perspective view of a plunger body of the measurement control system of Figure 4.
FIGURE 7 is a perspective view of the activation system of Figure 4.
FIGURE 8A is a sectional view of the measurement control system and activation system of Figure 4 in a first or "closed" position.
FIGURE 8B is a sectional view of the measurement control system and activation system of Figure 4 in a second or "open" position.
FIGURE 9 is a side view of an embodiment of an activation system, pressurized chamber, and first pressure regulation system of the second embodiment of a gas mixture apparatus.
FIGURE 10 is a sectional view of the activation system, pressurized chamber, and first pressure regulation system of Figure 9 in a first position.
FIGURE 11 is a sectional view of the activation system, pressurized chamber, and first pressure regulation system of Figure 9 in a second position.
FIGURE 12 is a sectional view of components including a mixing chamber and second pressure regulation system of a second embodiment of a gas mixture apparatus.
FIGURE 13 is an enlarged sectional view of the mixing chamber and second pressure regulation system of Figure 12.
FIGURE 14 is an enlarged sectional view of the mixing chamber and second pressure regulation system of Figure 12 with an additional attachment.
FIGURE 15A is a perspective view of a metering dial of an embodiment of a measurement control system.
FIGURE 15B is a sectional view of a metering dial of an embodiment of a measurement control system.
FIGURE 16 is a perspective view of a plunger body of an embodiment of a measurement control system.
FIGURE 17 is a perspective view of components of an embodiment of an activation system.
FIGURE 18 is a sectional view of a measurement control system and activation system in a first, "initial", or "pre-activation" position showing operation of an interlock mechanism.
FIGURE 19 is a sectional view of a measurement control system and activation system in a second or "open" position showing operation of an interlock mechanism.
FIGURE 20 is a sectional view of a measurement control system and activation system in a third or "closed" position showing operation of an interlock mechanism.
FIGURE 21 is a sectional view of a measurement control system and activation system in a first, "initial", or "pre-activation" position showing operation of the latch.
FIGURE 22 is a sectional view of a measurement control system and activation system in a second or "open" position showing operation of the latch.
FIGURE 23 is a sectional view of a measurement control system and activation system in a third or "closed" position showing operation of the latch.
FIGURE 24 is an enlarged view of an embodiment of an activation system, pressurized chamber, and a storage member pressure regulation system.
FIGURE 25A is a sectional view of the embodiment of an activation system, pressurized chamber, and a storage member pressure regulation system of Figure 24 in a first, "initial", or "pre-activation" position.
FIGURE 25B is an enlarged view of the embodiment of an activation system, pressurized chamber, and a storage member pressure regulation system of Figure 25A.
FIGURE 26A is a sectional view of the embodiment of an activation system, pressurized chamber, and a storage member pressure regulation system of Figure 24 in a second or "open" position.
FIGURE 26B is an enlarged view of the embodiment of an activation system, pressurized chamber, and a storage member pressure regulation system of Figure 26A.
FIGURE 27A is a sectional view of the embodiment of an activation system, pressurized chamber, and a storage member pressure regulation system of Figure 24 in a third or "closed" position.
FIGURE 27B is an enlarged view of the embodiment of an activation system, pressurized chamber, and a storage member pressure regulation system of Figure 27A.
FIGURE 28 is an enlarged view of the embodiment of an activation system, pressurized chamber, and a storage member pressure regulation system of Figure 25A illustrating in more detail an embodiment of a storage member.
FIGURE 29 is an enlarged view of the embodiment of an activation system, pressurized chamber, and a storage member pressure regulation system of Figure 26A illustrating in more detail an embodiment of a storage member.
FIGURE 30 is a sectional view of an embodiment of a syringe body and syringe pressure regulation system.

### DETAILED DESCRIPTION OF THE PREFERRED EMBODIMENT

The following detailed description is merely illustrative in nature and is not intended to limit the embodiments of the subject matter or the application and uses of such embodiments. As used herein, the word "exemplary" means "serving as an example, instance, or illustration." Any implementation described herein as exemplary is not necessarily to be construed as preferred or advantageous over other implementations. Furthermore, there is no intention to be bound by any expressed or implied theory presented in the proceeding technical field, background, brief summary, or the following detailed description.

Certain terminology may be used in the following description for the purpose of reference only, and thus are not intended to be limiting. For example, terms such as "upper", "lower", "above", and "below" refer to directions in the drawings to which reference is made. Terms such as "proximal", "distal", "front", "back", "rear", and "side" describe the orientation and/or location of portions of the component within a consistent but arbitrary frame of reference which is made clear by reference to the text and the associated drawings describing the component under discussion. Such terminology may include the words specifically mentioned above, derivatives thereof, and words of similar import. Similarly, the terms "first", "second", and other such numerical terms referring to structures.

As used herein, the terms "front" and "distal" refer to the parts of the subject apparatus which are located further away from the user (e.g., surgeon) of the apparatus during an injection operation. As used herein, the terms "rear" and "proximal" refer to the parts of the apparatus which are located closer to the user (e.g., surgeon) of the apparatus during an injection operation.

### Apparatus For Mixing Two Gases

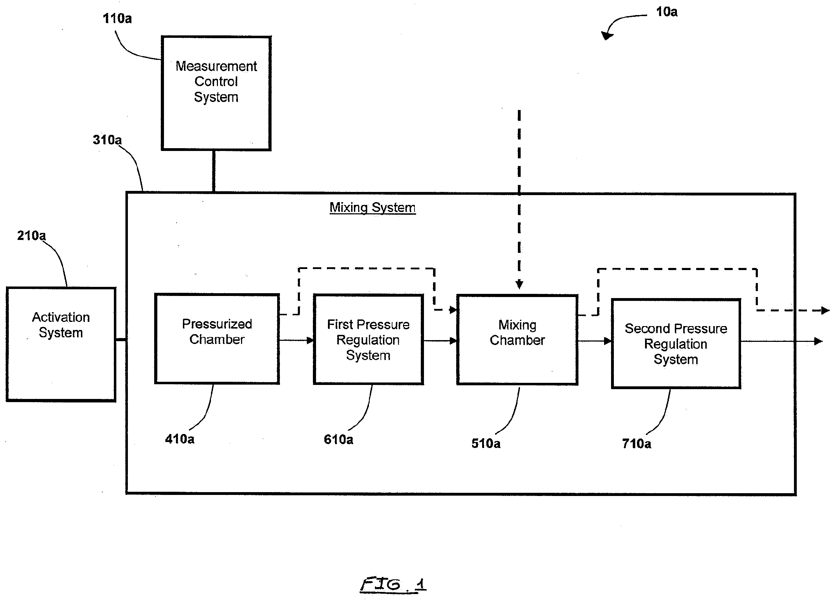

With reference to **FIG. 1****,** an embodiment of a gas mixture apparatus **10a** can comprise a measurement control system **110a,** an activation system **210a,** and a mixing system **310a** configured to create a mixture of two or more gases at a desired concentration ratio. The mixing system **310a** can include a pressurized chamber **410a** and a mixing chamber **510a.**

The mixing system **310a** can also include a pressure regulation system to enhance the operation of the mixing system **310a.** In some embodiments, the mixing system **310a** additionally includes a first pressure regulation system **610a** and a second pressure regulation system **710a.**

The measurement control system **110a** can be in the form of a metering mechanism operatively coupled to any and all devices contained within the mixing system **310a** to control certain aspects of the devices contained therein. In some embodiments, the measurement control system **110a** can be a variable and user-operable device such that aspects of the device can be modified by the user of the gas mixture apparatus **10a.** The activation system **210a** can be operatively coupled to the pressurized chamber **410a** in order to activate operation of the device and commence the mixing of gases within the mixing system **310a.**

The pressurized chamber **410a** can contain at least one of the two or more gases to be mixed within the mixing system **310a.** In some embodiments, the gas contained within the pressurized chamber **410a** can be at a pressure higher than surrounding ambient conditions. Additionally, the pressurized chamber **410a** can contain gases at concentrations different from that in the atmosphere. The pressurized chamber **410a** can be configured such that it is in fluid communication with the first pressure regulation system **610a.** In other embodiments, the pressurized chamber **410a** can be in direct fluid communication with the mixing chamber **510a.** The pressurized chamber **410a** can be configured such that it is internally contained within an injector apparatus. The pressurized chamber **410a** can also be configured such that it is external to the injector apparatus. The first pressure regulation system **610a** can be configured to maintain a pre-configured pressure differential between the pressurized chamber **410a** and the mixing chamber **510a.** The mixing chamber **510a** can be configured to receive gas from the pressurized chamber **410a** either directly or via the first pressure regulation system **610a.** In some embodiments, the mixing chamber **510a** can additionally be configured to receive a second gas to be mixed from outside the mixing system **310a** such as an external gas container or the atmosphere. The mixing chamber **510a** can be configured such that it is in fluid communication with the second pressure regulation system **710a** at a mixing chamber **510a** exit point. In other embodiments, the mixing chamber **510a** can be in direct fluid communication with the atmosphere at a mixing chamber exit point. Examples of each of these subsystems are described separately below.

In some embodiments, the measurement control system **110a** is configured to control concentrations of the gas within the gas mixture apparatus **10a.** In some embodiments, the measurement control system **110a** is operatively coupled with the mixing system **310a.** Preferably, measurement control system **110a** is operatively coupled with either the pressurized chamber **410a** or the mixing chamber **510a** such that the measurement control system **110a** can modify variable aspects of the pressure chamber **410a** and/or the mixing chamber **510a.** In some embodiments, the measurement control system **110a** is capable of controlling characteristics such as, but not limited to, the volume of gas contained within the mixing chamber **510a.** Other characteristics, such as pressure, are also contemplated as being controllable by the measurement control system **110a.** Preferably, the measurement control system **110a** is variable such that a user can be able to select a desired concentration ratio of gas that can be expelled from the gas mixture apparatus **10a.** This advantageously allows a user to have only a single gas mixture apparatus **10a** for a wide range of desired concentration ratios. As such, the measurement control system **110a** can include user-operable switches such as dials which vary the operation of components within the mixing system **310a** such as the pressurized chamber **410a,** the mixing chamber **510a,** the first pressure regulation system **610a,** and the second pressure regulation system **710a.**

The pressurized chamber **410a** is configured to store one or more gases within an interior space of the pressurized chamber **410a** for a period of time prior to mixing the two or more gases in the gas mixture apparatus **10a.** The conditions within the interior space is configured to be different than those of atmospheric conditions and therefore the interior space should generally reduce the release of such gases out of the interior space or reduce the entry of non-stored gases into the interior space until mixing of the two or more gases is to be performed.

In some embodiments, the one or more gases within the interior space are at a higher pressure than ambient atmospheric conditions. Additionally, the one or more gases can also be gases at concentrations different than those at ambient atmospheric conditions. In some embodiments, the interior space can be divided into separate subsections or portions for holding one or more gases. These separate portions of the interior space can therefore be kept at different pressures and/or different concentrations of gases.

In some embodiments, the gases can additionally be placed in different structural units within the interior space. Such structural units can be used to more effectively reduce the release of stored gases and/or reduce the entry of non-stored gases. In some embodiments, the stored gases of the pressurized chamber **410a** are pre-loaded from the time of manufacture. In other embodiments, it is contemplated that the contents of the pressurized chamber **410a** can be loaded by a user of the gas mixture apparatus **10a.** For example, the stored gases can be contained in a removable cartridge-like device which can advantageously facilitate the replacement of such gases.

In some embodiments, the activation system **210a** is configured to activate the operation of the gas mixture apparatus **10a** and commence the process of mixing the two or more gases within the mixing system **310a.** As such, the activation system **210a** is operatively coupled to the mixing system **310a** and can be coupled to both the mixing chamber **310a** and the pressurized chamber **410a.** The activation system can cause the pressurized chamber **410a** to activate and release gases contained therein into the mixing chamber **510a.** In some preferred embodiments, the activation system **210a** can cause the pressure within the pressurized chamber **410a** to increase such that the first pressure regulation system **610a** is activated thereby allowing fluid flow from the pressurized chamber **410a** into the mixing chamber **510a.** The activation system **210a** can include a device configured to activate a separate portion of the pressurized chamber **410a** that contains higher pressure gas than the remainder of the pressurized chamber **410a** such that the pressure within a separate section of the pressurized chamber **410a** increases. In a preferred embodiment, the activation system **210a** can cause a sealed device within the mixing chamber **510a** to burst thereby increasing the pressure throughout the pressurized chamber **410a.** In such embodiments, the activation system **210a** can include a puncturing device capable of bursting the seal. Use of an activation system **210a** provides advantages by allowing the gas mixture apparatus **10a** to potentially be pre-filled prior to use and safely stored.

The activation system **210a** can also be operably coupled to the mixing chamber **510a** allowing a user to manually vary certain aspects of the device. In some embodiments, the activation system **210a** can be used to modify the volume of the mixing chamber **510a.** The activation system **510a** can also be used to modify the pressure of the mixing chamber **510a.**

In some embodiments, the first pressure regulation system **610a** is configured to serve as a separation mechanism between both the pressurized chamber **410a** and the mixing chamber **610a.** The first pressure regulation system **610a** can activate upon reaching a pre-configured pressure differential between both the pressurized chamber **410a** and the mixing chamber **510a.** In some preferred embodiments, the first pressure regulation system **610a** can be comprised of at least one valve assembly. The valve assembly can open when pressure within a portion of the pressurized chamber **410a** is higher than the pressure in the mixing chamber **510a.** The valve assembly can be a check valve, clack valve, non-return valve, or one-way valve. Such valves can also include ball check valves, diaphragm check valves, swing check valves, stop-check valves, lift-check valves, in-line check valves, and duckbill valves. Other pressure regulation mechanisms can also be used. Additionally, it is contemplated that first pressure regulation system **610a** can also be activated by other means other than pressure differentials across the system **610a.**

In some embodiments, the mixing chamber **510a** is configured to serve as a space within which the two or more gases can be mixed to obtain a desired concentration ratio of the gases. The mixing chamber **510a** can be such that the volume can be variable and adjustable based upon use of the activation mechanism. The mixing chamber **510a** can receive the gases to mix solely from the pressurized chamber or from gases already existing within the mixing chamber **510a.** The mixing chamber **510a** can also receive gases from secondary sources. In some embodiments, the mixing chamber **510a** can receive air from the atmosphere to mix with the gases received from the pressure chamber **310a** and/or gases already existing within the mixing chamber **510a.**

In some embodiments, the second pressure regulation system **710a** is configured to serve as a separation mechanism between both the mixing chamber **510a** and the surrounding atmosphere. The second pressure regulation system **710a** can activate upon reaching a pre-configured pressure differential between both the mixing chamber **510a** and the surrounding atmosphere. In some preferred embodiments, the second pressure regulation system **710a** can be comprised of at least one valve assembly. The valve assembly can open when pressure in the mixing chamber **510a** is higher than the pressure in the surrounding atmosphere. The valve assembly can be a check valve, clack valve, non-return valve, or one-way valve. Such valves can also include ball check valves, diaphragm check valves, swing check valves, stop-check valves, lift-check valves, in-line check valves, and duckbill valves. Other pressure regulation mechanisms can also be used. Additionally, it is contemplated that second pressure regulation system **710a** can also be activated by other means other than pressure differentials across the system **710a.**

### Operational Overview

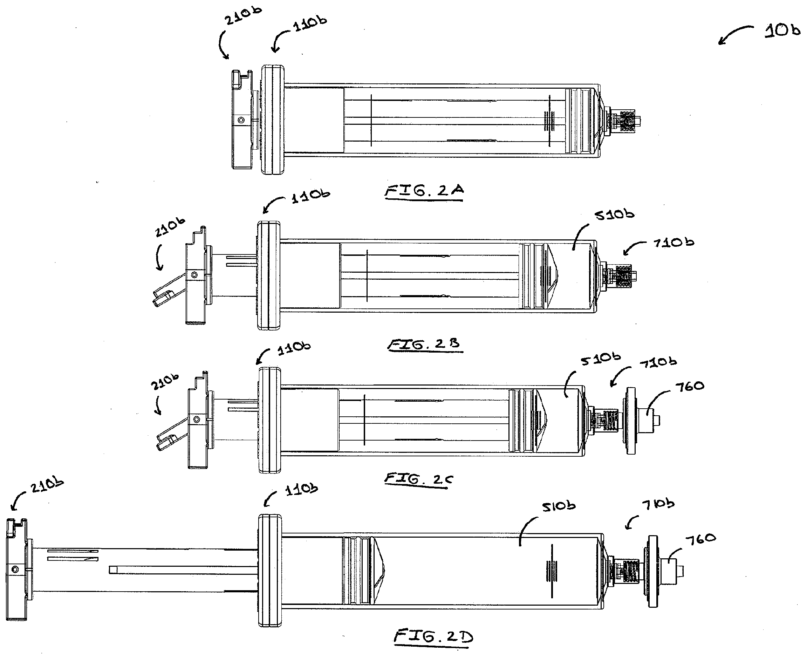

With reference to **FIGS. 2A-2D****,** the operation of an embodiment of a gas mixture apparatus **10b** is illustrated. With reference to **FIG. 2A****,** the apparatus **10b** can be in an initial phase with the activation system **210b** in a first or "closed" position. At this time, the user of the device can use the measurement control system **110b** to select a desired concentration of gas for the injectable volume. Once the selection has been made, the user can then move the activation system **210b** into a second or "open" position thereby causing the system to activate and commencing the mixing process.

During this first phase of operation, as shown in **FIG. 2B****,** gas contained within the pressurized chamber can be released and, in embodiments containing a first pressure regulation system, the first pressure regulation system can open in response to a change in pressure within the chamber. As such, fluid can flow from the pressurized chamber into the mixing chamber **510b** thereby causing an increase in the volume of the mixing chamber **510b.** However, due to components of the measurement control system **110b,** the mixing chamber **510b** can reach a first volume and cannot expand beyond this first volume. This first volume can be set based on the desired concentration of the injectable volume. During this first phase of operation, excess gas can also be bled from the mixing chamber **510b** via the second pressure regulation system **710b.** Once the mixing chamber has reached this first volume, the first phase of operation is complete and the second phase of operation begins.

During the second phase of operation, the mixing chamber **510b** can remain at the first volume while pressure within the mixing chamber **510b** is bled from the system via the second pressure regulation system **710b.** By overfilling the mixing chamber **510b** with the desired gas, and then bleeding off that gas, this helps to ensure that a significant amount of atmospheric gas within the mixing chamber **510b,** which may have been contained in the mixing chamber **510b** prior to activation, is substantially purged from the mixing chamber **510b** and displaced by the gas originally contained in the pressurized chamber. Once the pressure within the mixing chamber **510b** has reached a configured value based on the configuration of the second pressure regulation system 710b, bleeding of the gas within the mixing chamber **510b** ceases and the second phase of operation is complete.

During a third phase of operation, as shown in **FIG. 2C****,** an attachment **760** can be added to the system which can force the second pressure regulation system **710b** to remain open. This attachment can be a filter to remove bacteria to sterilize air, an infusion cannula or a syringe needle. This opening of the second pressure regulation system **710b** causes gas within the mixing chamber **510b** to reach ambient pressure. Once sufficient time has elapsed for the gas to reach ambient pressure, the user can then set the activation system **210b** to the first or "closed" position thereby unlocking the measurement control system **110b.** The user can then manually expand the volume of the mixing chamber **510b** to the injectable volume. In some embodiments, the measurement control system **210b** can stop expansion of the volume of the mixing chamber **510b** once the injectable volume is reached. Once the third phase is complete, the attachment can be removed such that the second pressure regulation system **710b** can isolate the mixed gas from the surrounding atmosphere to reduce or prevent dilution of the mixed gas.

One significant advantage of the operation of the apparatus **10b** is that the entire process can be performed by a single individual within the sterile field. Furthermore, the process is substantially automated such that the user need only set the measurement control system **210b** to a proper setting and the apparatus **210b** will automatically perform a substantial majority of the remaining steps. Additionally, the steps automatically performed by the apparatus **10b** are those which can normally be most-prone to error such as measuring proper volumes to achieve a desired concentration thereby significantly reducing the risk of obtaining an incorrect concentration of gas in the injectable volume. Inadvertent dilution of the gas with the surrounding atmosphere at the conclusion of the second and third phases of the operation can be reduced or prevented with the incorporation of the second pressure regulation system **710b.**

In other embodiments, a fewer or greater number of phases of operation can be performed. In some embodiments, only a single phase of operation can be performed. For example, the pressurized chamber **410a** can contain a gas at a pre-set concentration level. During the single phase of operation, the user can activate the apparatus **10b** such that a gas or fluid flows from the pressurized chamber **410a** and into a second chamber, such as the mixing chamber **510a,** until the chamber reaches a configured volume. The gas or fluid can also be expelled or bled off using a pressure regulation system until a desired pressure is achieved within the chamber. After expelling the gas, the apparatus **10b** can be ready for use. As should be apparent to one of skill in the art, in such an embodiment, little to no mixing may in fact be performed.

### System Overview

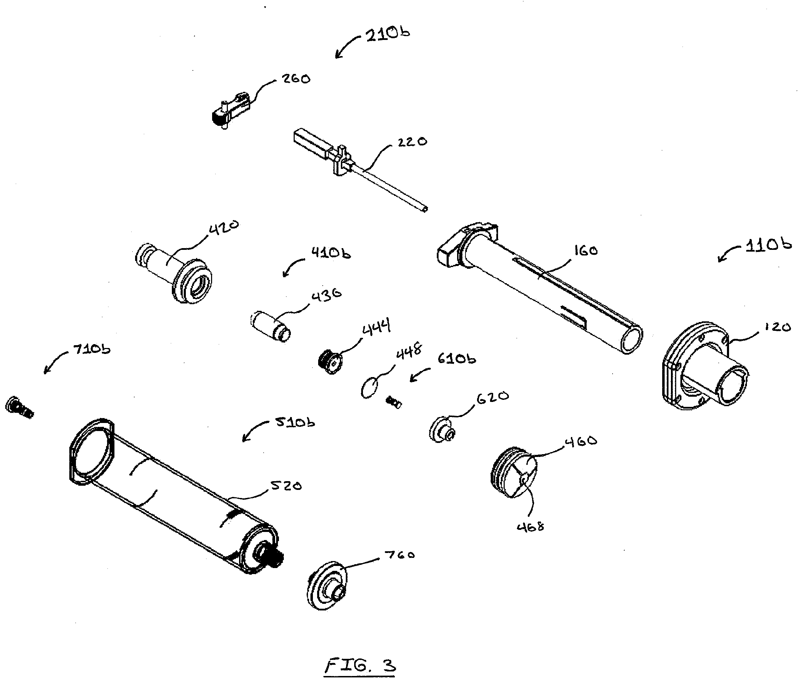

With reference to **FIG. 3****,** components of an embodiment of a gas mixture apparatus **10b** are shown which comprise a measurement control system **110b,** an activation system **210b,** a pressurized chamber **410b,** a mixing chamber **510b,** a first pressure regulation system **610b,** and a second pressure regulation system **710b.** The measurement control system **110b** can comprise a metering dial **120** and a plunger body **160** which can be inserted into the metering dial **120.** The activation system **210b** can comprise an actuation rod **220** and activation switch **260.** The activation system **210b** can be operatively coupled to the measurement control device **110b** to control the operation of the gas mixture apparatus **10b.** The activation system **210b** can be inserted into the plunger body **160.**

The pressurized chamber **410b** can be comprised of a housing **420,** a canister **436** containing a gas, a release mechanism **444** to release the gas contained within the canister **436,** a filter **448** to reduce the amount of non-gas or bacteria material flowing out of the housing **420,** and a plunger seal **460.** The mixing chamber **510b** can be comprised of a syringe body **520.** The first pressure regulation system **610b** can comprise a valve body and associated valve components. The second pressure regulation system **710b** can also comprise associated valve components.

### Measurement Control System and Activation System

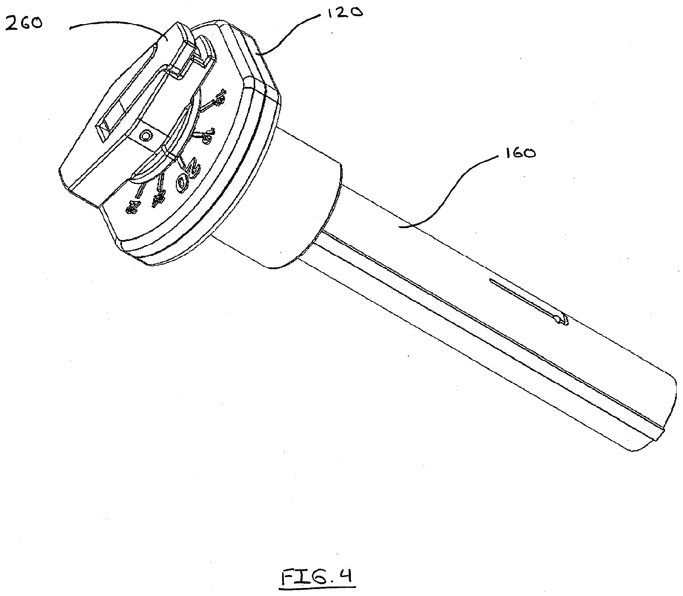

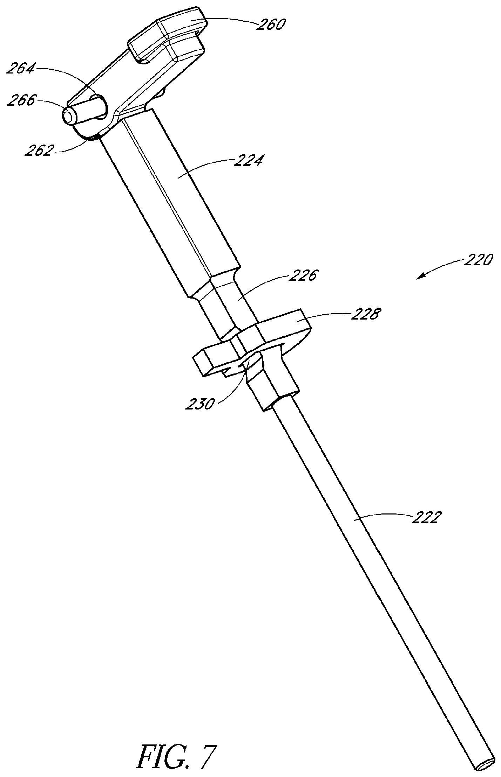

With reference to **FIG. 4****,** an embodiment of a combined measurement control system **110b** and activation system **210b** is shown. The measurement control system **110b** can comprise a metering dial **120** and a plunger body **160.** The activation system can comprise an actuation rod **220** (shown in **FIG. 7****)** and an activation switch **260.**

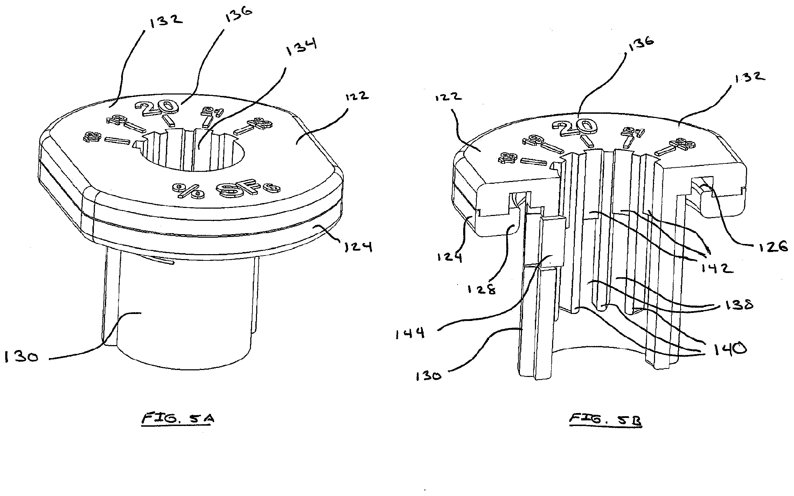

With reference to **FIGS. 5A and 5B****,** an embodiment of a metering dial **120** of the gas mixture apparatus **10b** is shown which is configured to allow a user of the apparatus **10b** to selectively vary the concentration of an injectable volume. The metering dial **120** is comprised of at least two structural components - a metering body **122** and a metering cap **124** which can be coupled to the metering body **122** so as to allow the metering dial **120** to be reversibly attached to another component of the apparatus **10b.** This can advantageously facilitate assembly of the apparatus and, in some embodiments which are reusable, can facilitate disassembly for resterilization. In some embodiments, the metering cap **124** can be reversibly attached to the metering body **122** using fasteners such as screws, rivets, clips, and other fastening mechanisms known in the art. Attachment of the metering cap **124** to the metering body **122** can form an annular slot **126** and an annular lip **128** such that the metering dial **120** can be attached to another component of the apparatus **10b.** For example, the annular slot **126** and annular lip **128** can correspond to a flange **526** located on the syringe body **520.**

The metering body **122** can have a generally cylindrical member **130** with a flange **132** at the top end and a channel **134** substantially centered on the cylindrical member **130** and running throughout the entire meter body **122.** Since the meter body **122** is configured to control the concentration of the gas in the injectable volume, the meter body **122** can include metering indicators **136** along a surface viewable by a user of the apparatus **10b** in a fully assembled state. In the illustrated embodiment, the metering indicators **136** are located on a top surface of the flange **132** although any location which can be viewed by the user can be used. The metering indicators **136** can provide the user of the device with information regarding the operation of the apparatus **10b.** In the illustrated embodiment, the metering indicators **136** show a range of numbers from 18, 19, 20, 21, and 22 corresponding to concentrations of sulfur hexafluoride (SF₆) which would be produced in the injectable volume if the apparatus **10b** is activated. As should be apparent to one of skill in the art, the ranges used can depend upon the gas used and the application for the gas. Furthermore, in some embodiments, this range can be further divided to provide enhanced control over the desired concentration.

The metering body **122** can have slots **138,** rails **140,** and variable stops **142** corresponding to the metering indicators **136.** In the illustrated embodiment, the metering body **122** has five separate slots **138** located along an inner surface of the channel **134** which correspond to the five integer values stated above. In other embodiments, the metering body **122** can have fewer or greater slots than the number of values provided by the metering indicators **136.**

Corresponding with each of these slots **138** are variable stops **142** which extend inwardly from the slots **138.** As illustrated above, these variable stops **142** can extend from the top surface of the flange **132** to a set distance towards the bottom end of the tubular body **130.** In some embodiments, the variable stops **142** need not extend from the top surface but instead are minor protrusions at set distances towards the bottom end of the cylindrical member **130.** These variable stops **142** are configured to interact with components contained in the plunger body **160** such as a latch **228,** or the plunger body **160** itself to control the expansion volume of the mixing chamber **510b** during a first and second phase of operation by limiting the rearward extension of the plunger body **160** during these phases (see **FIG. 2B****).** As such, the variable stops **142** extend different distances depending upon the concentration to which the stop **142** corresponds. For example, a concentration of 21 percent extends a lesser distance than a concentration of 20 percent. As such, when a concentration of 21 percent is chosen, the plunger body **160** can be allowed to extend rearwardly a greater distance thereby allowing a greater expansion of the mixing chamber **510b** during the first phase of operation. Therefore, as should be apparent, the variable stops **142** are used to control the first expansion volume of the first phase of operation.

On both sides of slots **138** are rails **140** which extend inward from an inner surface of the channel **134.** In some embodiments, the rails **140** extend inwardly from the inner surface of the channel **134** a greater distance than the variable stops **142.** The rails **140** can be configured to prevent the apparatus **10b** from switching to a different concentration value once the apparatus **10b** has been activated. This can be particularly important in applications where a specific concentration of gas can be necessary and any minor change in this value can have significantly adverse effects. In the illustrated embodiment, the rails **140** are configured to substantially reduce the likelihood that the plunger body **160** will rotate to a different variable stop **142** during at least the first two phases of operation. In certain embodiments, these rails can be removed if a constantly variable metering device is desired. In such an embodiment, the variable stop **142** could instead have a ramp shape rather than have multiple steps.

Metering body **122** can additionally include a ratchet pawl **144** along an inner surface of channel **134** which extends inwardly toward the center of the channel **134.** The ratchet pawl **144** can be hinged and configured such that the ratchet pawl **144** is movably deformable and provides resistance during deformation. This ratchet pawl **144** can correspond to features located on the plunger body **160** to facilitate proper orientation with respect to the selected concentration. Such a mechanism can additionally provide tactile feedback to a user of the device indicating that the proper alignment has been achieved. This tactile feedback can advantageously reduce the likelihood of activation in an improper orientation. Other types of feedback mechanisms and alignment mechanisms can also be used.

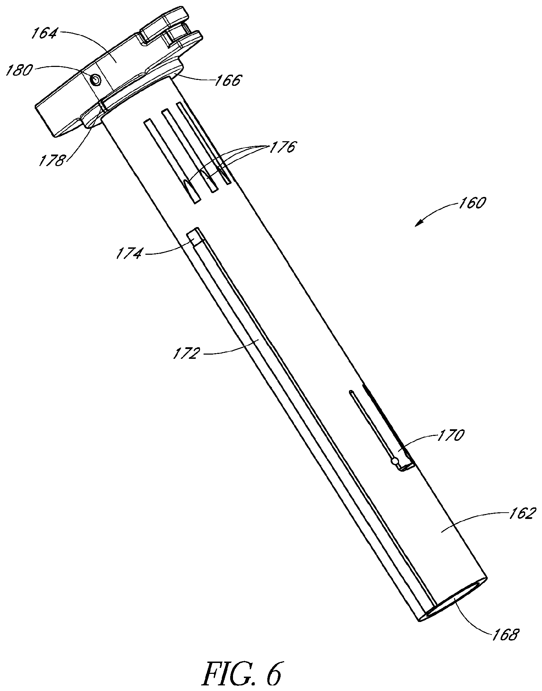

With reference to **FIG. 6****,** an embodiment of a plunger body **160** is shown which comprises a generally tubular frame **162,** a handle **164** at one end of the plunger body **160,** a selector ring **166** located therebetween, and a channel **168** centered on the tubular frame **162** and running throughout the entire length of the plunger body **160.** The tubular frame **162** is configured to be slidably translatable and partially slidably rotatable within the channel **134** of the metering dial **120.** The tubular frame **162** has a retention mechanism **170** in the form of a clip which is hingedly attached to the tubular frame **162.** The retention mechanism **170** can be configured to retain a component such as a housing **420** of the pressurized chamber **410b.** The retention mechanism **170** advantageously allows the component to be attached without the use of tools thereby facilitating the process of assembling the entire device. Additionally, the retention mechanism **170** can also be configured such that the component can be removed from the tubular frame **162** thereby allowing the apparatus **10b** to be reused or, in other embodiments which allow for reuse of the apparatus **10b,** facilitating the process of resterilization if such a process is used for the device. Other types of retention mechanisms can also be used in lieu of the clips shown in the illustrated embodiment and can include fasteners such as screws.

Tubular frame **162** can additionally comprise a guard **172** which extends outward from the outer surface of the tubular frame **162.** The guard **172** can run from the bottom end of the tubular frame **162** to a distance toward the top end of the tubular frame **162.** The guard **172** is configured to fit within the slots **138** and rails **140** located along the inner surface of the channel **134** of the metering body **122.** As such, the guard **172,** when positioned between the rails **140,** can prevent the plunger body **160** from rotating. This advantageously can prevent the plunger body **160** from moving to a different variable stop **142** after commencing the first phase of operation and thereby reduce the risk of an improper concentration in the injectable volume. The guard **172** is preferably sized such that, when the plunger body **160** is fully inserted, the guard **172** is only slightly below the rails **140** such that the plunger body **160** can rotate freely to different concentration values during the initial phase of operation (see **FIG. 2A****).** However, because the guard **172** is only slightly below the rails **140,** once extended a short distance, the guard **172** can become locked within the selected rail **140.** This positioning advantageously allows the guard **172** to lock shortly after activation of the apparatus **10b.** Furthermore, the guard **172** preferably extends outward from the tubular frame **162** only a sufficient distance such that it can contact the rails **140** but not enough such that it contacts the variable stops **142** located between the rails **140.** This can therefore allow the guard **172** to not be interfered by the variable stops **142** during operation.

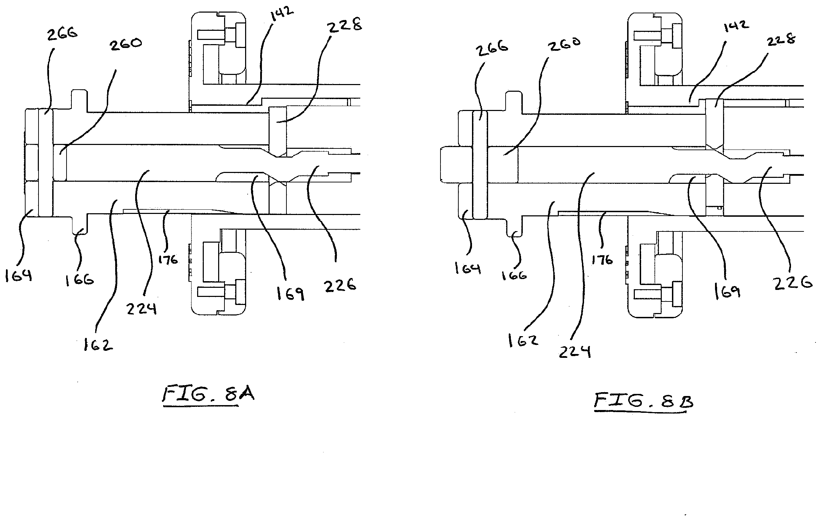

Tubular frame **162** can additionally comprise a latch aperture **174** configured to allow a latch **228** located on the activation rod **220** to protrude outward from the tubular frame **162.** The latch aperture **174** is preferably centered just above the top-most portion of the guard **172.** As will be discussed in detail below, in a first or "closed" position, the latch **228** can not extend beyond the guard **172** and thus would not contact a variable stop **142** (see **FIG. 8A****).** When in a second position, the latch **228** can extend outward from the tubular frame **162** beyond the guard **172** such that the latch **228** can contact the variable stops **140** thereby preventing further extension of the plunger body **160** while the latch is in the second position (see **FIG. 8B****).** In some embodiments, the latch aperture **174** can be placed such that, if the plunger body **160** is improperly oriented within the metering dial **120** during an initial phase of operation (shown in **FIG. 2A****),** the latch **228** can be prevented from extending outward into the second or "open" position by a rail **140** of the metering dial **120.** This can advantageously prevent the apparatus **10b** from activating when improperly oriented.

Tubular frame **162** can additionally include ratchet slots **176** in the form of cut-outs located along its outer surface. The ratchet slots **176** are configured to receive the ratchet pawl **144** of the metering body **122** thereby providing a mechanism for ensuring that the plunger body **160** is properly oriented within the metering body **122** by providing resistance against rotation when the pawl **144** is received within one of the ratchet slots **176.** Furthermore, advantageously, at each point where the ratchet pawl **144** is received within the ratchet slots **176,** a user of the apparatus **10b** can also receive tactile feedback when the plunger body **160** is properly oriented within the metering body **120.**

Selector ring **166** can be an annular protrusion extending from the outer surface of the tubular frame **162.** The selector ring **166** can additionally include a selector indicator **178** which can take the form of a minor protrusion located on the selector ring **166.** Selector indicator **178** can correspond to the metering indicators **136** located on the metering body **122** to indicate the concentration level that will be obtained when the plunger body **160** is oriented in that position. Such a system can advantageously provide a user of the device with easily viewed information regarding the selected concentration level. The selector indicator **178** can advantageously be colored to facilitate use of the apparatus **10b.**

The handle **164** can extend in two opposite directions in a radial direction from the longitudinal axis of the tubular frame **162.** IIandle **164** can be shaped such that a user of the apparatus **10b** can contact the handle **164** and use the handle to either further extend the plunger body **160** rearward and out of the apparatus **10b** or further depress the plunger body **160** frontward into the apparatus **10b.** Handle can additionally include a pin aperture **180** for receiving a coupling mechanism for the activation switch **260.** The activation switch **260** can thereby rotate about the coupling mechanism in order to operate the actuation rod **220** located within the plunger body **160.**

With reference to **FIG. 7****,** an embodiment of an activation system **210b** is shown which comprises an actuation rod **220** and an activation switch **260.** The actuation rod **220** has a generally elongate body with an actuator pin **222** at a first end, an actuator stem **224** at a second end, and a latch movement portion **226** located in an intermediate portion. The actuator pin **222** is configured to be received within a housing **420** of the pressurized chamber **410b** and activate the release of gas contained therein when in a second or "open" position. The actuator stem **224** is configured to abut and follow the contoured surface **262** of the activator switch **260.** The actuator stem **224** is also preferably shaped such that the cross-sectional profile matches the cross-sectional profile in a top portion of the channel **169** (as shown in **FIG. 8****)** located near the handle **164** of the plunger body **160.** Preferably, the cross-sectional profile is not substantially circular such that the actuator rod **220** is substantially prevented from rotating within the channel **168** of the plunger body **160.** The latch movement portion **226** is shaped such that the latch **228** is translated when the latch **228** slidably translates along the latch movement portion **226** of the actuation rod **220.** As such, the latch **228** has an aperture **230** which has a cross-sectional shape similar to that of the cross-sectional shape of the latch movement portion **226.**

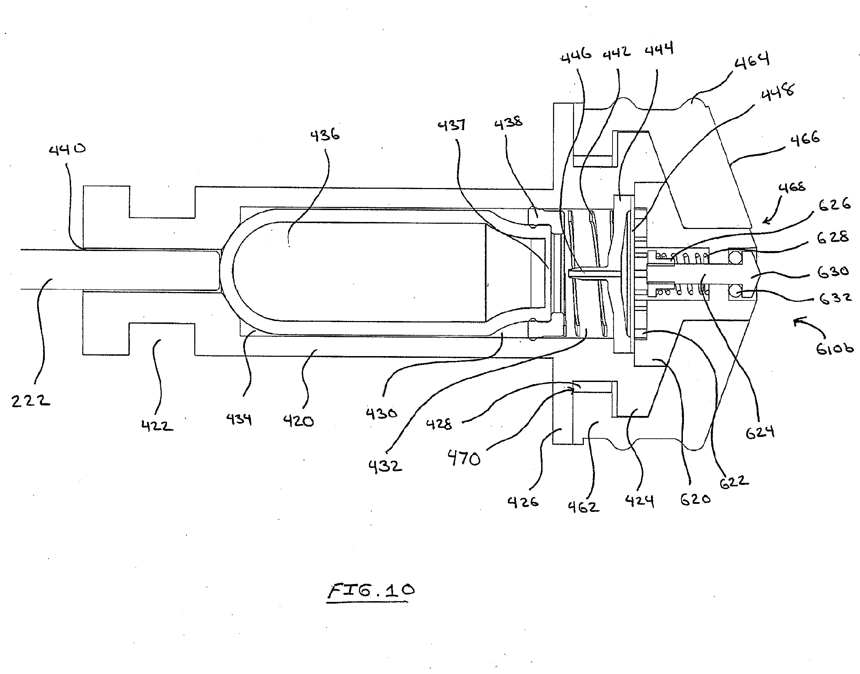

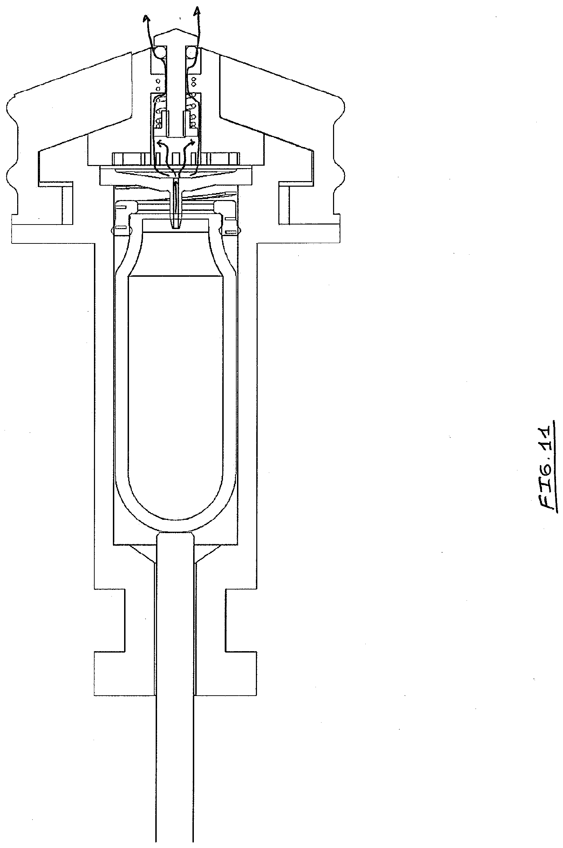

The activator switch **260** is configured to translate the actuator rod **220** through the plunger body **160** and through the housing **420** of the pressurized chamber **410b** to activate the release of gas contained therein. As such, the activator switch **260** can be a cam with a contoured profile **262** along the surface configured to contact the actuator stem **224.** Activator switch can also have an aperture **264** configured to receive a pin **266** such that the activator switch **260** can rotate about the pin **266.** In the illustrated embodiment, the activator switch **260** is shown in a first or "closed" position. In this first position, the distance between the pin **266** and the contoured surface **262** in contact with the actuator stem can be a reduced distance such that the actuator rod remains in a first or "closed" position. However, when rotated about the pin **266** to a second or "open" position, the distance between the pin **266** and the contoured surface **262** in contact with the actuator stem **224** can be an increased distance thereby translating the actuator rod **220** to a second or "open" position further into the housing **420** of the pressurized chamber **410b.** As will be discussed in greater detail with respect to **FIGS. 10** and **11** below, movement into the second or "open" position can be configured to release gas in the pressurized chamber **410b.** The activator switch **260** can preferably be any type of switch that can remain in a first or second position without the user needing to maintain the switch in that position. In the illustrated embodiment, a rotating lever is used. Other switches can also be used such as a screw, latch, spring loaded pin, or any other switch known in the art.

With reference to **FIGS. 8A** and **8B****,** an illustration of the operation of the activation system **210b** is shown which includes some components of the measurement control system **110b** and the activation system **210b.** As shown here, the latch **228** is contained within the latch aperture **174** such that the latch cannot translate toward a front end or rear end of the plunger body **160.** As such, when the actuator rod **220** translates in a frontward or rearward direction, the latch **228** must follow the profile of the latch movement portion **226** of the actuator rod **220.** As such, this provides the advantage of coupling movement of the latch **228** in the second position when the activator switch **260** and thus the actuator rod **220** are in a corresponding second position. Furthermore, because movement of the latch **228** is coupled with movement of the other activator switch **260** and actuator rod **220,** if the latch **228** is prevented from moving into the second position, the activator switch **260** and activator rod **220** are also prevented from moving into the second position. Note that, as described above, while in the second or "open" position, the latch **228** can protrude from the plunger body **160** thereby restricting extension of the plunger body **160** as shown in **FIG. 8B****.**

### Pressurized Chamber and First Pressure Regulation System

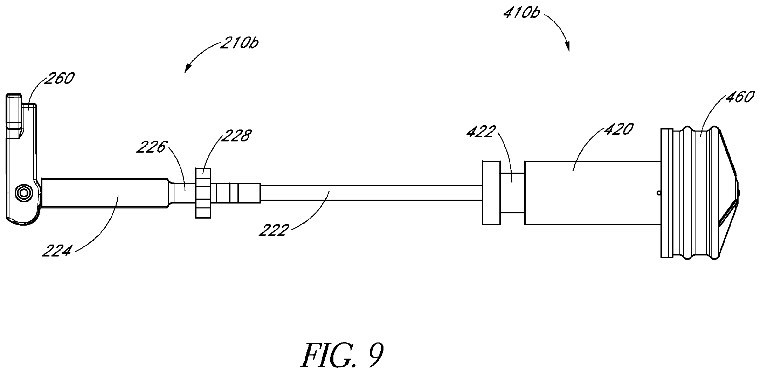

With reference to **FIG. 9****,** an embodiment is shown including some components of both the activation system **210b,** the pressurized chamber **410b** of the mixing system **310b,** and the first pressure regulation system **610b** of the mixing system **310b.** As illustrated, the pressurized chamber **410b** can have a housing **420** with an annular slot **422** located near a first end of the housing **420.** The annular slot **422** can be configured to receive the retention mechanism **170** located on the plunger body **160.** Housing can also have a plunger seal **460** located at a second end of the housing **420.** The plunger seal **460** is configured to provide an airtight seal for defining the mixing chamber **510b.**

With reference to **FIG. 10****,** which is a sectional view of the pressurized chamber **410b** and the first pressure regulation system **610b.** The housing **420** has a generally cylindrical body with an annular slot **422** located at the first or rearward end and a conical or frusto-conical surface **424** located at the second or frontward end corresponding to the shape of the plunger seal **460.** Housing **420** can additionally be shaped such that it has an annular protrusion **426** and an annular slot **428** configured to receive a lip **462** of the plunger seal **460.** This configuration advantageously ensures that the plunger seal **460** remains connected to the housing **420** and forms a seal to prevent the leakage of any gas contained in the housing body **420.** It can be preferable that the lip **462** of the plunger seal **460** fit snugly within the annular slot **428** of the housing **420** to provide an enhanced seal. An interior space **430** is substantially enclosed by the housing **420** and can be separated into a first separate portion **432** and a second separate portion **434.** Contained within the second separate portion **434** of the housing **420** can be a third separate portion in the form of a structural unit such as a canister **436.** This canister can contain the gases for mixing into the mixing chamber **510b.** Provision of the gases in a canister is advantageous as it facilitates manufacturing of the apparatus **10b** as it can allow the canisters to be manufactured separately from other components of the pressurized chamber **410b.** In some embodiments where the apparatus **10b** is reusable, cartridges can be replaced.

The canister **436** has a first or rearward end in contact with the actuator pin **222** and a sealed second or frontward end **437.** At one end of the canister **436** is a seal **438** which substantially reduces leakage of any gas from the first separate portion **432** to the second separate portion **434.** This advantageously reduces the likelihood of gases from leaking out of the actuator aperture **440** and out of the apparatus **10b.**

The housing can also include a biasing mechanism **442,** such as a spring, which exerts a force on the seal in a direction away from the second end of the housing **420.** In the illustrated embodiment, the biasing mechanism **442** is located in the first separate portion **432.** This reduces the likelihood of the canister **436** moving into the first separate portion **432** and potentially releasing the gas contained therein without having been activated by the user. Furthermore, biasing mechanism **442** can also provide a counter-force against activation such that a user can not accidentally activate the device. It is preferable that the biasing mechanism **442** be configured to exert a sufficient force such that, after the first and second phases of operation are complete and the activation switch **160** is returned to a first or "closed" position, the biasing mechanism **442** exerts sufficient force such that actuator rod **220** is returned to its first or "closed" position thereby causing the latch **228** to return to its first or "closed" position. Once latch **228** returns to its first or "closed" position, the extension of the plunger body **160** is no longer limited and the third phase of operation can commence. If the biasing mechanism **442** does not exert sufficient force on the actuator rod **220,** entering into the third phase of operation could be made significantly more difficult.

Housing can also have a release mechanism **444,** such as a needle or a pilot tip as illustrated in this embodiment of the apparatus **10b,** which can be configured to puncture the sealed second end **437** of the canister **436** to release gas into the first separate portion **432** through the release mechanism **444** due to a channel **446** running axially through release mechanism **444.** Due to the high pressure in the first separate portion **432,** the first pressure regulation system **610b** can open allowing the gas to escape to the front of the plunger seal **460** and into the mixing chamber **510b.** In some embodiments, a filter **448** can be placed along the flow path such that there is a reduced likelihood of foreign materials entering into the mixing chamber **510b.** This can be particularly important when the gas can be placed into areas highly sensitive to the presence of foreign materials such as bodily cavities. The presence of foreign materials can cause infection or other harm. In some embodiments, the filter **448** can be configured to filter out bacteria to sterilize the air.

Plunger **seal 460** is configured to partially define the injectable volume of the mixing chamber **510b** by creating a seal for the mixing chamber **510b.** Plunger seal**460** can have a generally cylindrical body with annular protrusions **464** configured to contact an inner surface of the mixing chamber **510b** and a conical or frustoconical face **466** at a frontward end. The frustoconical face **466** can additionally comprise an aperture **468** centered about the cylindrical body configured to receive components of the first pressure regulation system **610b.** Furthermore, the body can also have an opening **470,** defined by the lip **462,** on the rearward end configured to receive the housing **420.**

With continued reference to **FIG. 10****,** an embodiment of the first pressure regulation system **610b** is shown in a first or "closed" position. The first pressure regulation system **610b** can comprise a valve body **620** comprising multiple apertures **622** at one end, a valve stem **624** running through the valve body **620** with a seat **626** at a rear end configured to contact the biasing mechanism **628** and a head **630** at a front end configured to contact a sealing ring **632.** During operation, the biasing mechanism **628** can exert a biasing force against the seat **626** in a rearward direction such that the head **630** is biased against the sealing ring **632** and valve body **620** thereby reducing or preventing the flow of gas through the valve body **620** and ultimately into the mixing chamber **510b.** Due to the orientation of the biasing mechanism **628,** the first pressure regulation system **610b** remains closed until pressure within the pressurized chamber **410b** exceeds a threshold value. This threshold value can be configured by changing the amount of force necessary to compress the biasing mechanism **628.**

With reference to **FIG. 11****,** an embodiment of the first pressure regulation system **610b** is shown in an "open" position during the first and second phase of operation. During these phases, pressure within the pressurized chamber **410b** can exceed the pressure within the mixing chamber **510b.** In some preferred embodiments, the difference in pressure is substantial. Due to this pressure differential, sufficient force is placed upon the valve components causing the biasing mechanism **628** to be overcome thereby allowing gas to flow out of the valve body **620** and into the mixing chamber **510b.**

This configuration for the first pressure regulation system **610b** is advantageous due to the multiple phases of operation of the apparatus **10b.** During the first and at least part of the second phase of operation, the pressure differential causes the valve to remain open. However, once the pressure differential is insufficient to overcome the threshold value, the valve remains in a closed position preventing any additional gas from flowing into the mixing chamber and potentially disrupting the calculated prcssurcs/conccntrations.

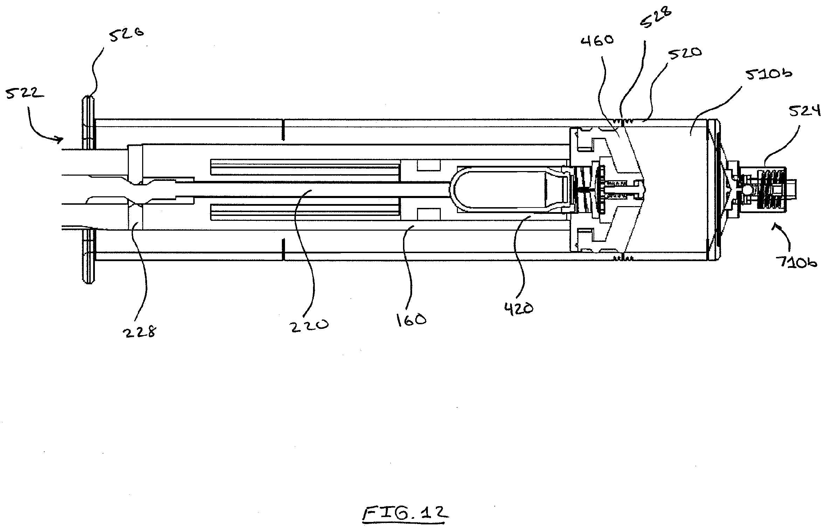

With reference to **FIG. 12****,** an embodiment of a mixing chamber **510b** is shown comprising a syringe body **520,** a second pressure regulation system **710b,** and various components of the above-mentioned systems. Syringe body **520** has a cylindrical body, an aperture **522** at the rear end, and a threaded nozzle **524** at the front end. Syringe body also has flange **526** configured to be engaged with the metering device **120.** The mixing chamber **510b** can be defined by the inner walls of the syringe body **520** and the plunger seal **460.** Furthermore, the syringe body can include indicators **528** along its outer surface corresponding to a chosen concentration. These indicators **528** can advantageously provide visual confirmation to the user of the selected concentration.

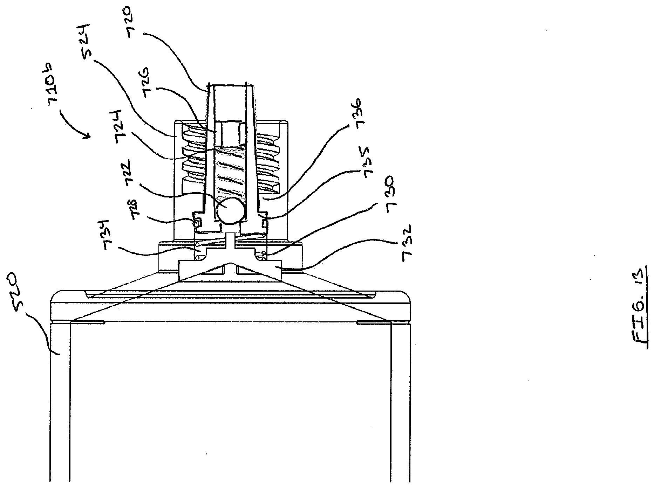

With reference to **FIG. 13****,** an embodiment of the second pressure regulation system **710b** is shown comprising a valve body **720** which can include a ball **722,** a biasing mechanism **724,** a seat **726,** and a sealing mechanism **728.** The second pressure regulation system **710b** can also comprise a second biasing mechanism **730** and a pin actuator **732.** The valve body **720** can be translatable within the interior space **734** near the nozzle **524** of the syringe body 520. In some embodiments, due to the second biasing mechanism **730,** the valve body **720** is translated such that a flange **735** of the valve body **720** is pressed against the inner lip **736** of the nozzle **524.** Furthermore, biasing mechanism **724** can seal flow through the valve body **720** until a sufficient force is placed on the ball **722** to overcome the biasing force. This can occur when the pressure differential between the mixing chamber **510b** and the atmosphere is beyond a threshold value.

During operation, the second pressure regulation system **710b** is opened during a first and second phase of operation due to the increased pressure contained in the mixing chamber **510b.** Once the pressure differential is insufficient to cause valve body **720** to open, the second phase of operation is complete and the user can move proceed to the third phase of operation.

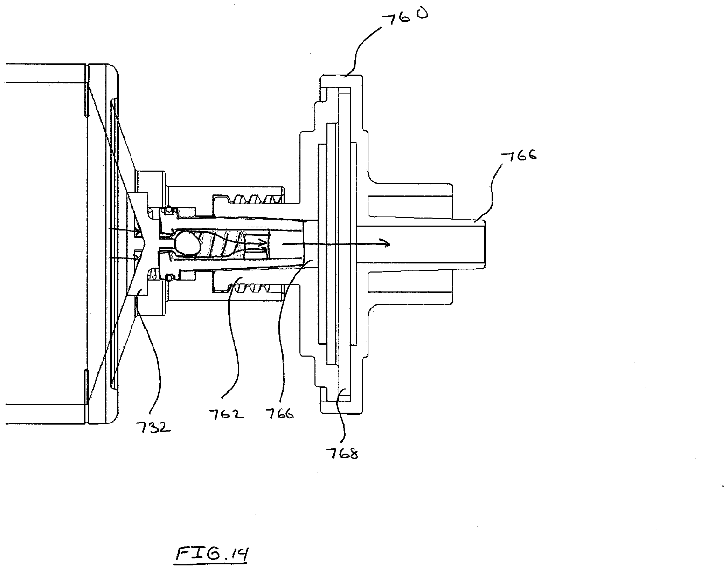

With reference to **FIG. 14****,** an embodiment of the second pressure regulation system **710b** is shown with an attachment **760** comprising a filter. The attachment **760** has a first open end **762** with a flange **764** configured to engage with the threads on the interior of the threaded nozzle **524,** a second open end **766,** and a filter clement **768** located therebetween. As such, gas can pass from the first open end **762** to the second open **766** and advantageously be filtered in the process which reduces the risk of any harmful materials enter the mixing chamber **510b.** In some embodiments, the inner surface of the first open end **762** tapers when moving towards the second open end **766** such that the shape corresponds to the shape of valve body **720.** As the attachment **760** is threaded into the threaded nozzle **524,** the attachment **760** engages the valve body **720** and translates the valve body **720,** against the biasing force of the second biasing mechanism **730** towards the rear end of the syringe body **520.** This causes the ball **722** to engage the pin actuator **732** thereby causing the valve body **720** to open allowing gas within the mixing chamber **510b** to reach ambient pressure. This configuration is advantageous as it allows the mixing chamber **510b** to be further expanded at ambient pressure and simultaneously filtering air drawn into the mixing chamber **510b.** In this position, the third phase of operation can therefore be performed. Once the third phase of operation is completed, the attachment **760** can be removed. Due to the force of the second biasing mechanism **730,** the valve body **720** can be translated away from pin actuator **732** such that the valve body **720** remains closed until a user decides to inject the gas.

### Embodiment of Measurement Control System and Activation System

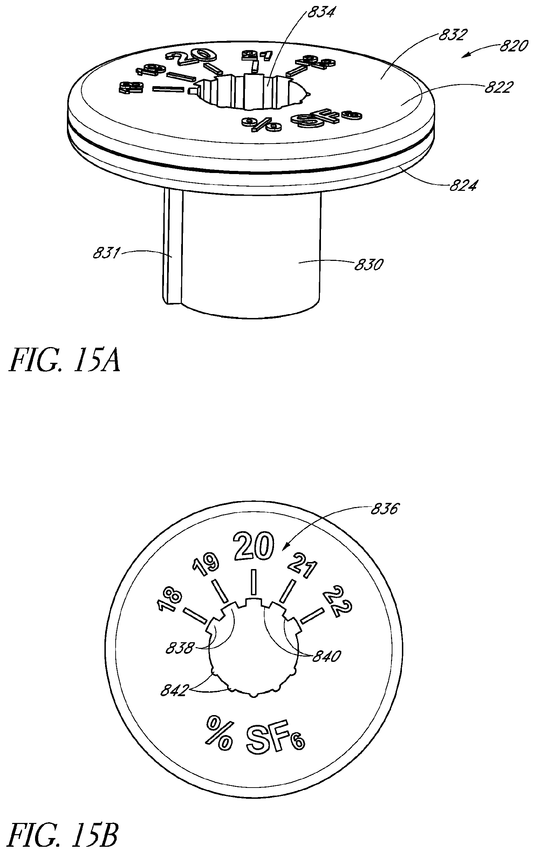

**FIGS. 15-31** illustrate additional embodiments of components of a measurement control system of the apparatus.

**FIGS. 15A** and **15B** illustrate an embodiment of a metering dial **820** which can be configured to allow a user of the device to select a concentration of fluid for an injectable volume. Similar to other embodiments, the metering dial **820** can include two components such as a metering body **822** and a metering cap **824** which can be removably attached to the metering body **822.** As with other embodiments of a metering dial and similar metering devices, such as metering dial **120,** the removable attachment can advantageously facilitate the assembly of the apparatus. Furthermore, in some embodiments, the removable attachment can allow for disassembly such that the apparatus can be reduced to individual components to facilitate resterilization of some or all of the components of the apparatus. As with other embodiments, the metering cap **824** can be reversibly attached to the metering body **822** using fasteners such as screws, rivets, clips, and other fastening mechanisms known in the art. In other embodiments, the metering body **822** and metering cap **824** can be irremovably attached using devices and methods such as adhesives, welding, and the like. Such embodiments can provide an advantage of reducing the likcihood of tampering. Attachment of the metering cap **824** to the metering body **822** can form an annular slot and an annular lip such that the metering dial **820** can be attached to another component of the apparatus. In some embodiments, the annular slot and the annular lip can correspond to corresponding features, such as a flange and lip, located on a syringe on which the metering dial **820** is placed.

With continued reference to **FIG. 15A** and **FIG. 15B****,** the metering body **822** can have a generally cylindrical member **830** with a flange **832** located at top portion of the metering body **822.** The metering body **822** can include a channel **834** substantially centered on the cylindrical member **830** and running throughout the entire metering body **822.** In some embodiments, the generally cylindrical member **830** can be sized and shaped to be received within a channel of another component of the apparatus. For example, in some embodiments, the metering body **822** can be received within a channel of a syringe to which the metering dial **820** is attached. In some embodiments, such as that illustrated in **FIG. 15A****,** the generally cylindrical member **830** can include additional surface features, such as an increased diameter portion **831,** which can potentially be keyed to the device into which it is inserted.

As with other embodiments of metering dials or similar metering mechanisms, this embodiment can also include metering indicators **836** located along a surface of the metering body **820.** In this illustrated embodiment the metering indicators **836** are located on a top surface of the flange **832** although any other viewable location can be used such as, for example, along the perimeter portion of the flange **832.** In the illustrated embodiment, the metering indicators **836** show a range of numbers from 18, 19, 20, 21, and 22 corresponding to concentrations of sulfur hexafluoride (SF₆) which can be produced in an injectable volume of the assembly. As should be apparent to one of skill in the art, the ranges used can depend upon the gas used and the application for the gas. In some embodiments this range can be further divided to provide greater precision and control over the desired concentrations.

As with other embodiments of metering dials and other metering mechanisms, the metering body **822** can have slots **838,** rails **840,** and variable stops corresponding to the metering indicators **836.** As more clearly shown in **FIG. 15B****,** the metering body **822** can have five separate slots **838** located along an inner surface of the channel **834** which correspond to the five metering positions 18, 19, 20, 21 and 22. In other embodiments, the metering body **822** can have fewer or greater slots than the number of values provided by the metering indicators **836.** Corresponding with each of these slots **838** can be variable stops which extend inwardly from the slots **838.** These variable stops can extend from the top surface of the flange **832** to a set distance towards the bottom end of the generally cylindrical member **830.** As should be appreciated by one of skill in the art, the variable stops need not extend from the top surface. For example, in some embodiments, the variable stops can be protrusions at set distances towards the bottom end of the tubular body **830.**

The operation of the variable stops of the illustrated embodiment of the metering dial **820** can be similar to that of other embodiments of metering dials and metering mechanisms. The variable stops can be configured to interact with components contained within the plunger body **860,** such as a latch **928** or similar protruding structure, to control expansion of a chamber for an injectable volume during at least some phases of operation. In some embodiments, the variable stops can perform this task by limiting the rearward extension of the plunger body **860** during different phases. As such, the variable stops extend different distances depending upon the concentration to which the stop corresponds.

With continued reference to **FIGS. 15A** and**15B****,** both sides of slots **838** can be bounded by rails **840** which extend inwardly from an inner surface of the channel **834.** In some embodiments, the rails **840** can extend inwardly from the inner surface of the channel **834** a greater distance than the stops. The rails **840** can be configured to prevent the apparatus from switching to a different concentration value once activated. In the illustrated embodiment, the rails **840** are configured to substantially reduce the likelihood that the plunger body **860** will rotate to a different variable stop during at least the first two phases of operation. In certain embodiments, the rails **840** can be removed if a constantly variable metering device is desired. In such circumstances other mechanisms can be used to prevent or otherwise significantly reduce the likelihood that a different concentration value will be chosen after the device has been activated.

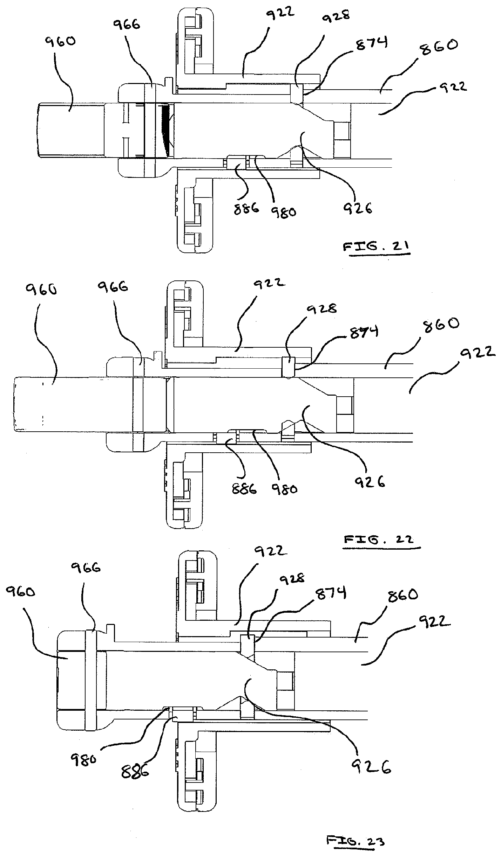

As illustrated more clearly in **FIG. 15B****,** metering body **822** can additionally include along an inner surface of the channel **834** notches, indentations, divots, recesses, or similar structures **842** located along an inner surface of the channel **834** opposite the slots **838** and rails **840.** In other embodiments, the notches **842** can be located at other suitable locations on the metering dial **820.** These notches **842** can correspond to features located on other components of the apparatus to form a ratcheting mechanism. For example, the notches **842** can correspond to a ratcheting member **886** (shown on **FIGS. 21-23****)** located on the plunger body **860.** As such, the ratcheting mechanism can be configured to advantageously provide a user with tactile feedback when the plunger body **860** has been rotated to a selectable concentration. As such, a user of the device can be less likely to accidentally have the plunger body **860** in an inoperable position when the gas assembly is activated. Furthermore, the ratcheting mechanism can also provide a threshold resistance against rotation from one concentration to a second concentration. In such embodiments, the ratcheting mechanism can thereby advantageously reduce the likelihood of unintentional rotation from one concentration to a second concentration. Other types of feedback mechanisms and alignment mechanisms can also be used to provide this tactile feedback.

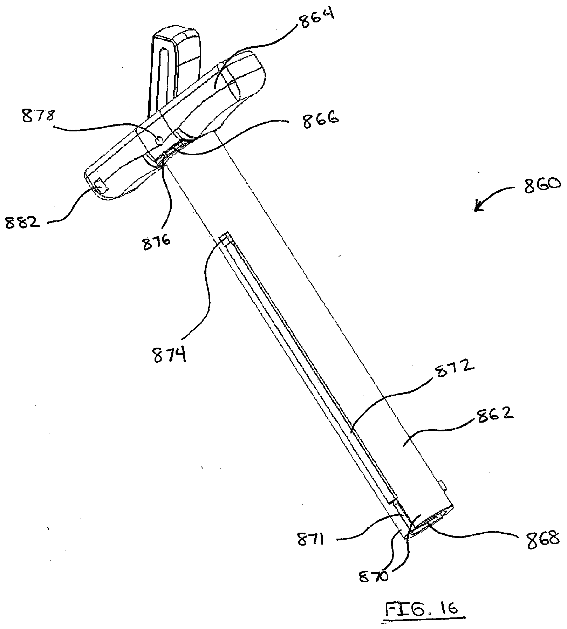

With reference to **FIG. 16****,** an embodiment of a plunger body **860** is shown which can include a generally tubular frame **862,** a handle **864** at one end of the plunger body **860,** a selector member **866** located there between, and a channel **868** centered on the tubular frame **862** which can run throughout the entire length of the plunger body **860** or which can run throughout at least a part of the length of the tubular frame **862.** The tubular frame **862** can be configured to slidably translate and slidably rotate within a channel of a metering dial.

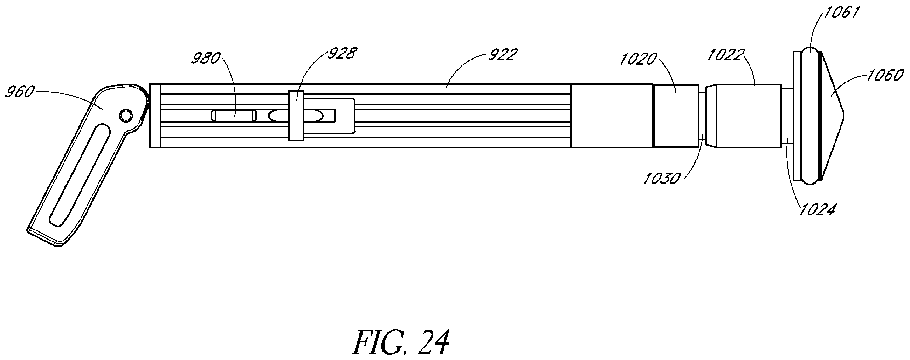

The tubular frame **862** can include retention wings or clips **870** located at an end opposite of the handle **864.** As shown in the illustrated embodiment, the retention wings **870** can be partially cylindrical protrusions separated by two or more cutouts or slits **871.** As such, depending on the material used, the retention wings **870** can be bent outwardly when receiving a component within the channel **868.** In some embodiments, the retention wings **870** can each include a semi-annular lip along an interior surface of the retention wings **870** which corresponds to an annular slot of a component inserted within the channel **868.** For example, in some embodiments, the semi-annular lip can correspond to an annular slot **1024** located on a second housing member **1022** (see **FIG. 24****).** As such, the retention wings **870** can allow for a snap fit assembly of multiple components of the device thereby facilitating assembly and also potentially allowing for disassembly for purposes of reuse and/or resteriilzation. Other fastening mechanisms and methods can also be used to connect the components to the plunger body **860** including fasteners such as screws, adhesives, welding, and other similar mechanisms and methods known in the art.

With continued reference to **FIG. 16****,** the tubular frame **862** can include a guard **872** which can extend outwardly from the outer surface of the tubular frame **862.** In some embodiments, the guard **872** can run from the bottom end of the tubular frame **862** to a distance toward the top end of the tubular frame **862,** such as, for example, up to and adjacent the latch aperture **874.** In other embodiments, such as that illustrated in **FIG. 16****,** the guard **872** can be sized so as to not extend to an end surface of the tubular frame **862** but instead extends only to the cutout **871** of the retention wings **870.** Similar to other embodiments of the plunger body, such as plunger body **160,** the guard **872** can be configured to fit within slots and rails of the metering dial. In other embodiments, other forms of metering devices can be used and the guards **872** can be configured to correspond to similar structural features on such devices.

The guard **872,** when positioned between the rails **840,** can prevent or substantially reduce the likelihood that the plunger body **860** will rotate after activation. This advantageously can prevent or reduce the likelihood of the plunger body **860** rotating during phases of operation which may cause an erroneous concentration of fluid in the injectable volume. The guard **872** can be sized such that, when the plunger body **860** is fully inserted within the channel **834,** the guard **872** can be slightly below the rails **840** such that the plunger body **860** can rotate freely to select different concentration values while in a first, "initial," or "pre-activation" position. However, because the guard **872** is only slightly below the rails **840,** once extended a short distance, the guard **872** can become positioned between the selected rails **840.** This positioning advantageously allows the guard **872** to lock shortly after activation of the apparatus. Furthermore, the guard **872** can extend outwardly from the tubular frame **860** only a sufficient distance to contact the rails **840** but not sufficiently outwardly to contact variable stops or similar features located between the rails **840.**

With continued reference to **FIG. 16****,** the tubular frame **862** can include a latch aperture **874** configured to allow a latch **928** located on the activation rod **920** and contained within the channel **868** to protrude outwardly from the tubular frame **862.** As shown in the illustrated embodiment, the latch aperture **874** can be centered just above the topmost portion of the guard **872.** In other embodiments, the latch aperture **874** can also be located at different positions along the tubular frame **862** and can contain more than one latch aperture if multiple latches are used.

As described in greater detail below, in a first, "initial", or "pre-activation" position, the latch **928** can be sized so as to not extend beyond the guard **872** and thus not contact a variable stop or similar structure. When in a second or "open" position, the latch **928** can extend outwardly from the tubular frame **862** beyond the guard **872** such that the latch **928** can contact the variable stops or similar structures thereby preventing or significantly reducing the likelihood of further extension of the plunger body **860** while the latch is in the second position. As with other embodiments of the plunger body **860,** in some embodiments the latch aperture **874** can be placed such that, if the plunger body **860** is improperly oriented within the metering dial **820** during an initial or "pre-activation" phase of operation, the latch **928** can be prevented from extending outwardly into the second or "open" position by a rail **840** of the metering dial **820.** Furthermore, similar to other embodiments of latch mechanisms, this can also prevent or at least substantially reduce the likelihood that a user will be able to operate the activation switch **960** thereby preventing or substantially reducing the likelihood that a user will activate the apparatus when in an inoperable position.

Selector member **866** can be a protrusion extending from the outer surface of the tubular frame **862.** The selector member **866** can additionally include a selector indicator **876** which can take the form of a minor protrusion located on the selector ring **866.** Selector indicator **876** can correspond to the metering indicators **836** located on the metering body **822** to indicate the concentration level that will be obtained when the plunger body **860** is oriented in that position when activated.

With continued reference to **FIG. 16****,** the handle **864** can extend in two opposite directions in a radial direction from the longitudinal axis of the tubular frame **862.** The handle **864** can be shaped such that a user can grip the handle **864** and use the handle to either further extend the plunger body **860** rearwardly, for example, to increase the volume contained in the apparatus or further depress the plunger body **860** frontwardly, for example, to reduce the volume contained in the apparatus and eject the injectable volume. The handle **864** can additionally include a pin aperture **878** for receiving a coupling mechanism, such as a coupling pin, for an activation switch **960.** The activation switch **960** can thereby rotate about the coupling mechanism in order to operate an actuation rod **920** which can be located within the plunger body **860.**

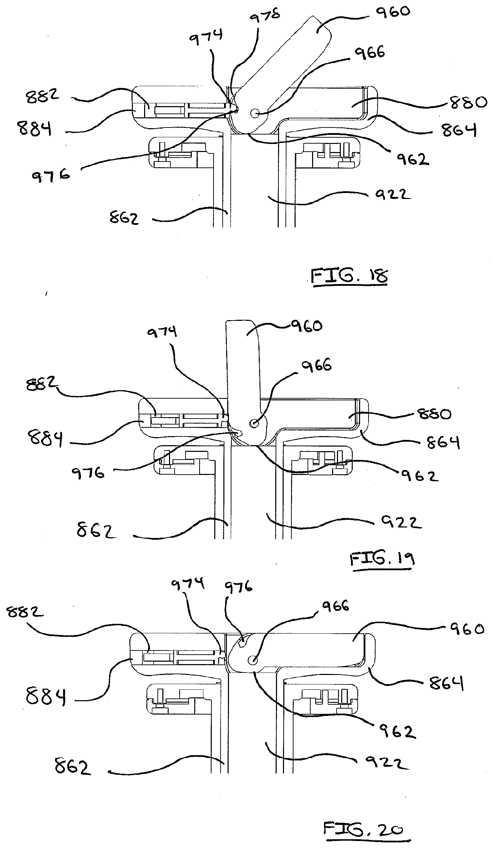

As will be described in more detail with respect to the operation of an interlock mechanism shown in **FIGS. 18-20****,** the handle **864** can additionally include a recess **880** configured to receive the activation switch **960.** The recess **880** can be sized such that, when the activation switch **960** is in a third or "closed" position, the activation switch **960** is fully contained within the recess **880.** Furthermore, the handle **864** can additionally include an interlock aperture **882** and an interlock channel **884** configured to receive an interlock member **970** of an interlock mechanism.

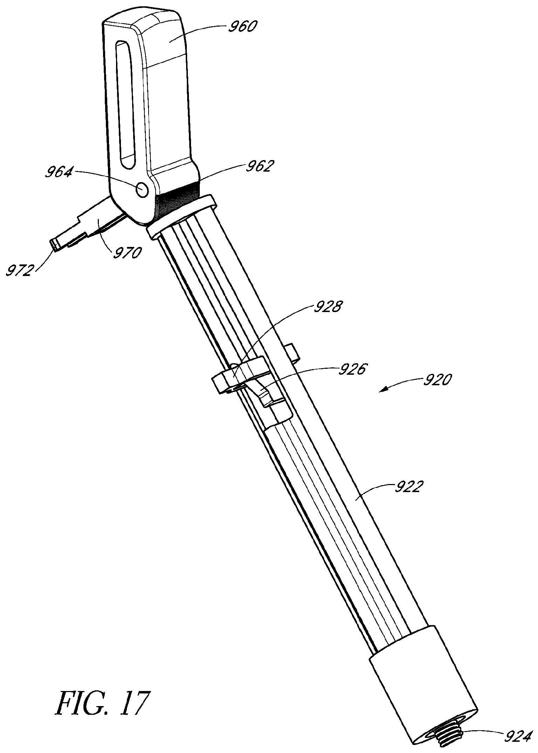

With reference to **FIG. 17****,** an embodiment of an activation system is shown which can include an actuation rod **920** and an activation switch **960** which can be used to control the operation of the apparatus. As shown in the illustrated embodiment, the actuation rod **920** can include an actuator body **922** with a generally cylindrical shape. The actuator body **922** can be configured to extend through part of the channel **868** of the plunger body **860.** In other embodiments, the actuator body **922** can be lengthened or shortened depending on the length of other components contained within the channel **868.** In some embodiments, the actuator body **922** can have other cross-sectional shapes such as circles, ovals, ellipses, quadrilaterals, or other polygons. Additionally, the actuator body **922** can differ in cross-sectional shape along different portions of the actuator body **922.** For example, as shown in the illustrated embodiment, the actuator body **922** can have a circular cross-sectional shape along a first portion of the actuator body **922** and a "+" cross-sectional shape in a second portion. Similar to other embodiments of the actuation rod, the actuator body **922** can be configured to abut and follow a contoured surface **962** of the activation switch **960** at a first end of the actuator body **922.** In some embodiments, the actuator body **922** can be translated within the channel **868** of the plunger body **860** when the activation switch **960** is rotated as a result of the contoured surface **962.**

In some embodiments, such as that illustrated in **FIG. 17****,** the actuation rod **920** can include a rod biasing member or mechanism **924** such as a helical spring or any other similar mechanism. The rod biasing member **924** can be configured such that it applies a linearly increasing force as the rod biasing member **924** is compressed. In other embodiments, the rod biasing member **924** can be configured such that it applies an exponentially increasing force as the rod biasing member **924** is compressed such that the force becomes significantly greater as the rod biasing member **924** is compressed. As shown in the illustrated embodiment, the rod biasing member **924** can be a helical spring received in a recess of the actuator body **922.** The rod biasing member **924** can be releasably fastened to the actuator body **922** such that the rod biasing member **924** can be removed for purposes of disassembly. In other embodiments, the rod biasing member **924** can be permanently fastened to the actuator body **922.** In yet other embodiments, the rod biasing member **924** can be not connected to the actuator body **922** and can be retained within the recess as a result of being placed between two components such as the actuator body **922** and a first housing member **1020.**

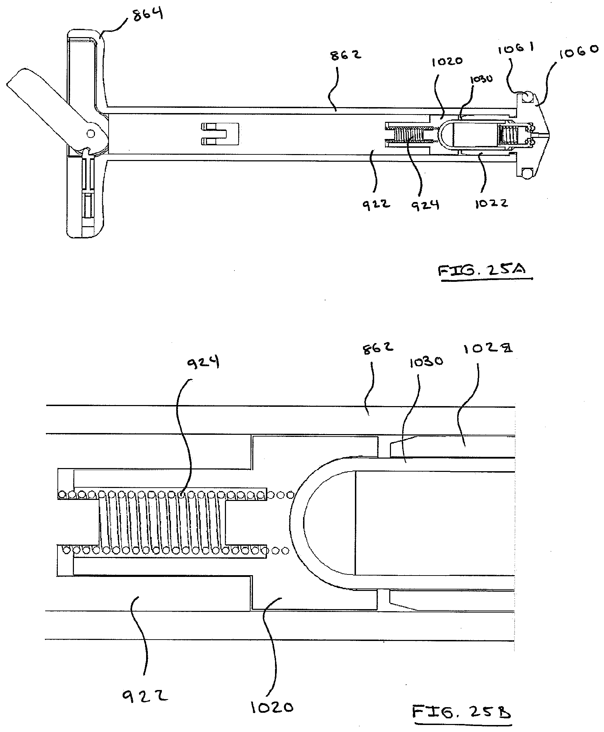

As is described in further detail below with respect to the operation of an embodiment of the activation system shown in **FIGS. 25-27****,** the rod biasing member **924** can be configured to provide a biasing force against a housing member **1020.** This biasing force can be configured such that it can exceed a threshold force to activate the release of gas from a pressurized chamber within the apparatus. In addition, the rod biasing member **924** can also be configured to provide a biasing force against the actuator body **922** such that, when the activation switch **960** is moved into different positions, the actuator body **922** will translate in a direction that will keep the actuator body **922** in contact with at least a portion of the activation switch **960** such as the contoured portion **962.**

The actuation rod **920** can include a latch movement portion **926** located between a first end and second end of the actuator body **922.** Similar to the latch movement portion of other embodiments of the actuation rods, latch movement portion **926** can be used to translate a latch **928** located thereon such that the latch **928** can protrude from or retract from an aperture or similar structure located on the plunger (e.g., latch aperture **874** located on the plunger body **860).**

With continued reference to **FIG. 17****,** an activator switch **960** can be configured to translate the actuator rod **920** through the plunger body **860** towards the first housing member **1020** to activate a mechanism for releasing the gas contained therein. As such, the activator switch **960,** like the activator switch of other embodiments, can be a cam with a contoured profile **962** located along the surface configured to contact the actuator body **922.** Activator switch **960** can additionally include an aperture **964** configured to receive a pin (not shown in **FIG. 17****)** such that the activator switch **960** can rotate about the pin. It should be appreciated by a person of skill in the art that the activation switch **960** can preferably be any type of switch that can remain in a first, second, or more positions without the user needing to maintain the switch in that position. In the illustrated embodiment, a rotating lever is used. Other switches can also be used such as a screw, latch, spring loaded pin, or any other switch known in the art.

With reference to **FIG. 18****,** the activator switch **960** is shown in a first, "initial", or "pre-activation" position. For example, this can be a position prior to a first phase of operation. In this first position, the distance between the pin **966** and the contoured surface **962** in contact with the actuator body **922** can be a first distance such that the actuator body **922** is located at a first distance from the end of the tubular frame **862** of the plunger body **860.**

As shown in **FIG. 19****,** in some embodiments, the activator switch **960** can be rotated towards a more vertically oriented position, a second or "open" position, in which the distance from the pin **966** to the contoured surface **962** in contact with the actuator body **922** can be a second distance such that the actuator body **922** is located at a second distance from the end of the tubular frame **862** of the plunger body **860.** This can correspond to the position of the activation switch **960** during a first and second phase of operation. In some embodiments, the second distance can be greater than the first distance. As will be described in more detail with respect to **FIGS. 25-27****,** this can cause the actuator body **922** to translate towards the first housing member **1020** of the pressurized chamber. This translation can activate the release of fluid or gas contained in the pressurized chamber.

As shown in **FIG. 20****,** in some embodiments, the activation switch **960** can also be rotated towards a more horizontally-oriented position, a third or "closed" position, in which the distance from the pin **966** to the contoured surface **962** in contact with the actuator body **922** can be a third distance such that the actuator body **922** is located at a third distance from the end of the tubular frame **862** of the plunger body **860.** This can correspond to a third phase of operation and/or a final phase prior to injection of the injectable volume into a patient. This third distance can be less than or equal to the first and/or second distances. In some embodiments, rotation towards the third position can cause the actuator body **922** to translate away from the first housing member **1020** of the pressurized chamber such that no fluid or gas is released from the pressurized chamber.

With reference back to **FIG. 17****,** an interlock mechanism can be included to control and limit the movement of the activation switch **960.** As shown in the illustrated embodiment, the interlock mechanism can include an interlock member **970** such as a pawl having interlock wings or clips **972** located at one end and an interlock portion **974** (shown in **FIGS. 18-20****)** located at a second end. The interlock clips **972** can be configured to be received within interlock apertures **882** or indentations, recesses, or other similar mechanisms to retain the interlock clips **972** located in the handle **864.**

With reference again to **FIGS. 18-20****,** an illustration of the operation of an embodiment of the interlock mechanism is provided. **FIG. 18** is an illustration of the interlock mechanism and activation switch **960** in the first, "initial", or "pre-activation" position. As shown in the illustrated embodiment, the interlock member **970** can be sized and shaped to be received within an interlock channel **884** of the handle **864.** While in the first position, the interlock clips **972** can be biased inwardly by virtue of contact between the interlock clips **972** and the inner surfaces of the channel **884.**

As shown in the illustrated embodiment, the interlock portion **974** of the interlock member **970** can be received within a notch or indentation **976** located at an end of the activation switch **960.** The shape of interlock portion **974** and the notch or indentation 976 can be chosen such that, while in the first or "initial" position, the activator switch **960** can be prevented or restricted from rotating in a clockwise direction towards a horizontally-oriented position (i.e., the third or "closed" position) due to resulting interference between the interlock portion **974** and the activation switch notch **976.** Additionally, the shape of the interlock portion **974** can be chosen such that, in the first position, the activation switch **960** can rotate in a counter-clockwise direction towards a more vertically-oriented position (i.e., the second or "open" position). In some embodiments, such as that shown in the illustrated embodiment, the activation switch **960** can include a second contoured surface **978** configured to translate the interlock member **970** towards an opposite end of the handle **864** when the activation switch **960** is rotated from the first to the second position. In some embodiments, movement of the interlock member **970** within the interlock channel **884** towards an opposite end of the handle **864** results in the ends of the interlock clips **972** being translated towards the interlock apertures **882.** Upon reaching the interlock apertures **882,** the interlock clips **972** which were originally pre-biased inwardly while in the interlock channel **884,** expand outwardly such that the interlock clips **972** are received within the interlock apertures **882.** In some embodiments, the interlock member **970** can be prevented from translating back towards the activation switch **960** once received within the interlock apertures **882.** This can advantageously prevent or at least substantially reduce the likelihood that the interlock member **970** can reengage the activation switch **960** and restrict movement of the activation switch **960.**

**FIG. 19** is an illustration of the activation switch **960** and the interlock mechanism in a second or "open" position. As illustrated, the interlock clips **972** of the interlock member **970** have been received within the interlock apertures **882** such that the interlock member **970** can no longer translate back towards the activation switch **960.** As a result, a user of the device can rotate the activation switch **960** in a clockwise direction towards the third or "closed" position.

**FIG. 20** is an illustration of the activation switch **960** and the interlock mechanism in the third or "closed" position. In some embodiments, the activation switch **960** can be received within a recess **880** in the handle **864** and be flush with a top surface of the handle. Furthermore, the recess **880** can be sized and shaped to closely conform to the shape of the activation switch **960** such that a user of the device can have difficulty rotating the activation switch **960** into one of the prior two positions after the activation switch has been fully placed in the third position.

As such, the interlock mechanism advantageously controls the operation of the activation switch **960** such that a user of the device will not accidentally rotate the switch **960** in an improper position or in an improper order. Furthermore, because a user of the device may have more difficulty rotating the activation switch **960** from the third position to one of the prior two positions, there is a reduced likelihood that a user could potentially alter the concentration of the injectable volume after the final phase of operation. As such, the interlock mechanism advantageously serves as a safety mechanism for operation of the device. In other embodiments, other forms of interlock mechanisms may be used which may include the use of other fasteners, clips, or similar devices. A person of ordinary skill in the art would understand that other types of interlock mechanisms can also be used.

With reference to **FIGS. 21-23****,** an illustration of the operation of an embodiment of the activation system is shown. As shown in the illustrated embodiment, and similar to other embodiments, the latch **928** can be contained within the latch aperture **874** such that the latch can not translate toward a front end or rear end of the plunger body **860.** In such an embodiment, when the actuator rod **920** translates in a frontward or rearward direction, the latch **928** is configured to follow the profile of the latch movement portion **926** of the actuator rod **920.**

**FIG. 21** shows the embodiment in a first, "initial", or "pre-activation" position. As shown here, the latch **928** can be positioned such that it outwardly protrudes from the plunger body **860** sufficiently such that, if extended rearwardly, the latch **928** would contact a variable stop located on the metering body **922** and prevent any further extension. In other embodiments, when in the first position, the latch **928** can be configured so as to not outwardly protrude from the body **860** to prevent such extension. When moved to the second or "open" position, as shown in **FIG. 22****,** the latch **928** can sufficiently outwardly protrude from the plunger body **860** such that the latch **928** can contact the variable stop or similar structure located on the metering dial **820** thereby preventing any further rearward extension. When rotated to the third or "closed" position, as illustrated in **FIG. 23****,** the latch **928** can be sufficiently retracted within the latch aperture **874** such that the latch **928** no longer contacts the variable stop or similar structure located on the metering dial **820** thereby allowing the plunger body **860** to be further extended rearwardly.

With continued reference to **FIGS. 21-23****,** a ratcheting member **886** such as a pawl can be attached to the plunger body **860.** The ratcheting member **886** can be hinged and configured such that the ratcheting member **886** is movably deformable and provides resistance during deformation. The ratcheting member **886** can correspond to features located on the plunger body metering dial **820,** such as notches **842,** to facilitate proper orientation with respect to the selected concentration. In order to allow inward deformation of the ratcheting member **886,** the actuator body **924** can include a recess or indentation **980.** This recess **980** can be configured such that the ratcheting member **886** is allowed to inwardly deform only in the first and third positions whereas the ratcheting member **886** is restricted from deforming inwardly while in the second position. This can provide a means of reducing the likelihood that the plunger body **860** can be rotated during operation of the device.

### Embodiment of Pressurized Chamber

With reference to **FIG. 24****,** an embodiment of a pressurized chamber is shown along with components of an activation system. As illustrated, the pressurized chamber can have a two-part housing with a first housing member **1020** and a second housing member **1022** which are translatable with respect to each other. As shown in the illustrated embodiment, the two members **1020, 1022** can have a generally cylindrical shape such that some or all portions of the two members **1020, 1022** can be received within a channel **868** of the plunger body **860.** In some embodiments, the two members **1020, 1022** can be detached from one another to allow free translation of the two members **1020, 1022.** In other embodiments, the two-part housing can be attached while still allowing translation of the members **1020, 1022** with respect to each other. Such attachment can be used to increase the stability of the two members **1020, 1022.**

As shown in the illustrated embodiment and similar to other embodiments of the pressurized chamber, an annular slot **1024** can be located on the second housing member **1022.** In the illustrated embodiment, the annular slot **1024** is located at an end opposite the first housing member **1020** however other possible locations can be chosen. The annular slot **1024** can be sized and configured to receive the retention wings **870** of the plunger body **860** allowing the second housing member **1022** to be fastened to the plunger body **860** using a snap-fit connection. To facilitate insertion of the second housing member **1022** into the channel **868** of the plunger body **860,** the inserted end portion can be slightly tapered. In some embodiments, the second housing member **1022** can be removably attached to the plunger body **860** thereby allowing replacement of certain parts contained therein. For example, in some embodiments, a storage member **1030** or canister can be contained within the two-part housing. The two-part housing can also have a plunger end **1060** with a plunger seal **1061** such as a rubber o-ring configured to sealingly contact the syringe body **1120** and form a seal for defining a chamber to contain an injectable volume, such chamber potentially serving as a mixing chamber. Other types of sealing members can be used around the plunger end **1060** to form such a seal.

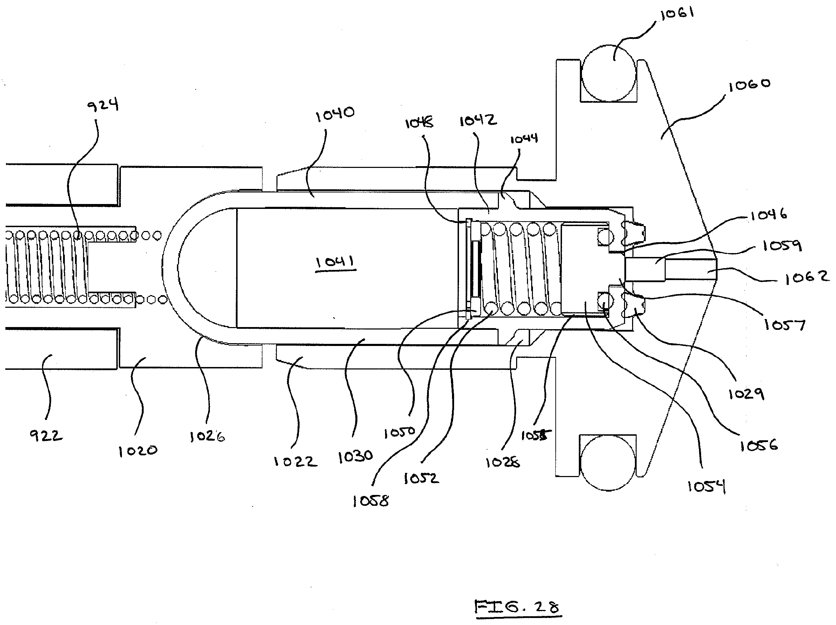

**FIGS. 25A** and **25B** are cross-sectional views of the embodiment shown in **FIG. 24** when the apparatus is in a first, "initial", or "pre-activation" position. As illustrated more clearly in **FIG. 25B****,** in the first position, the rod biasing member **924,** such as a helical spring can be in contact with both the actuator body **922** and the first housing member **1020;** however, the actuator body **922** may not be in direct contact with the first housing member **1020.** In the first position, the rod biasing member **924** can exert a force in a frontward direction upon the first housing member **1020** and a force in a rearward direction upon the actuator body **922** such that the actuator body **922** remains in contact with the activation switch **960.** In this position, the frontward force upon the first housing member **1020** can cause the first housing member **1020** to apply a force upon a storage member **1030** contained therein as the first housing member **1020** attempts to translate towards the second housing member **1022.** Preferably, in the first position, the force applied by the first housing member **1020** upon the storage member **1030** will be insufficient to translate the storage member **1030** towards the second housing member **1022** due to mechanisms contained in the storage member **1030** (as will be discussed in further detail in **FIGS. 28-29****).** As such, while in the first position, any gas or fluid contained within the storage member **1030** will remain contained within the storage member **1030.**

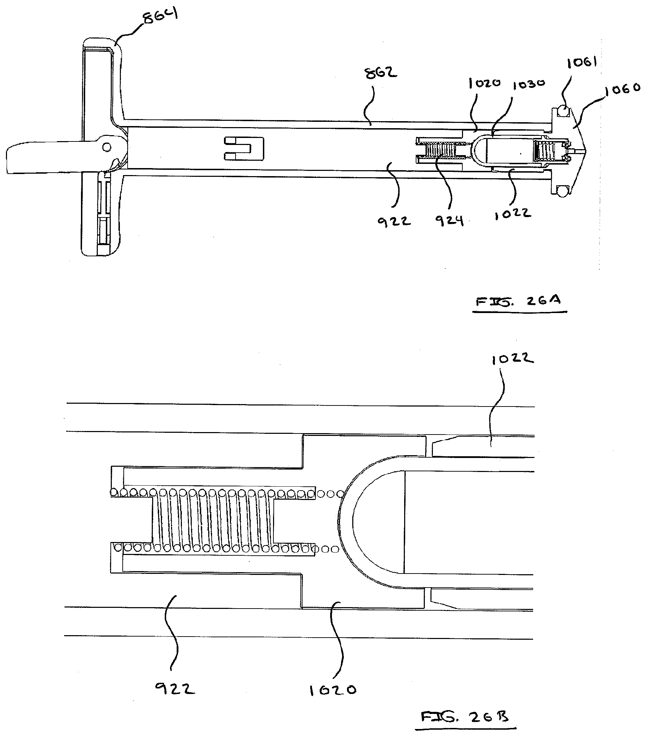

**FIGS. 26A** and **26B** are cross-sectional views of the embodiment shown in **FIG. 24** when the apparatus is in a second or "open" position. As illustrated more clearly in **FIG. 26B****,** while in the second position, both the actuator body **922** and the rod biasing member **924** can be directly in contact with the first housing member **1020.** Due to this direct contact, a more significant force can be applied to the first housing member **1020** such that the first housing member **1020** can translate in a frontward direction thereby causing the storage member **1030** to translate in a frontward direction. This frontward translation of the storage member **1030** can then activate the release of gas from the storage member **1030.** In other embodiments, the actuator body **922** need not directly contact the first housing member **1020** since, in such embodiments, the increase in force applied by the rod biasing member **924** due to compression of the rod biasing member **924** can be sufficient to cause the first housing member **1020** to translate in a frontward direction to cause the activation of the release of gas from the storage member **1030.**

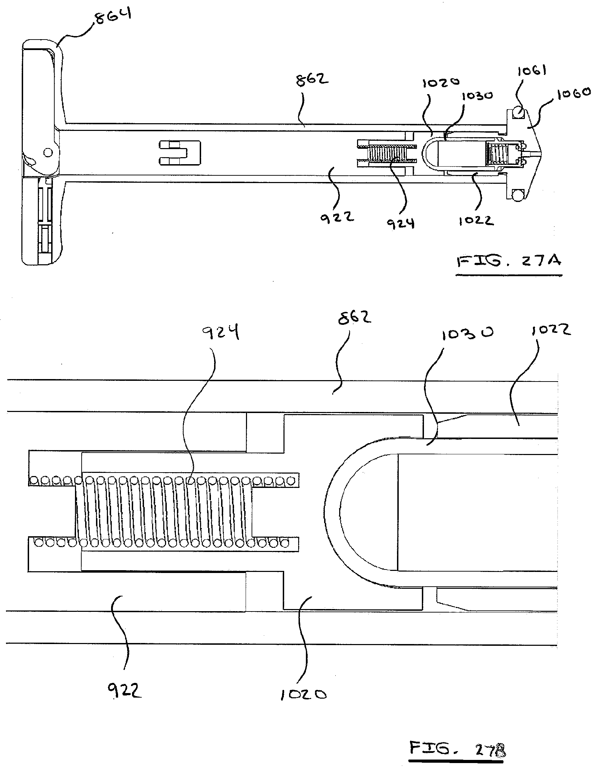

**FIGS. 27A** and **27B** are cross-sectional views of the second embodiment shown in **FIG. 24** when the apparatus is in a third or "closed" position. As illustrated in **FIG. 27B****,** while in the third position, the actuator body **922** may not be in contact with the first housing member **1020.** Furthermore, in some embodiments, due to the reduced distance between the pin **966** and the contoured surface **962,** the force exerted by the rod biasing member **924** on the actuator body **922** in a rearward direction can cause the actuator body **922** to translate towards the contoured surface **962** such that the actuator body **922** remains in contact with the activation switch **960.** This expansion of the rod biasing member **924** results in a reduction of force exerted by the rod biasing member **924** upon the first housing member **1020.** As a result of this reduced force, and as a result of other mechanisms located within the storage member **1030** or canister, the storage member **1030** can be restored to a closed state thereby preventing any additional gas from being released into the chamber to contain the injectable volume, which can also serve as a mixing chamber.

**FIG. 28** is a sectional view of an embodiment of a pressurized chamber. The first and second housing members **1020, 1022** have contained therein a storage member **1030** or canister, such as a microcylinder, which contains a fluid such as gas. In some embodiments, the second member **1022** has at an end opposite the first member **1020** a conical or frusto-conical surface forming the plunger end **1060.** In some embodiments, the second member **1022** and the plunger end **1060** form an integral unit. In other embodiments, the second member **1022** and the plunger end **1060** are separate units which can be attached using a variety of fastening devices and methods such as, but not limited to, fasteners such as screws and pins, retention clips, adhesives, welding, or the like. The plunger end **1060** can have an annular slot configured to receive a plunger seal **1061** such as a rubber o-ring to form a chamber for the injectable volume, which can also serve as a mixing chamber.

The first housing member **1020** can include a recessed portion **1026** or indented portion configured to contact and receive a first end of the storage member **1030.** The shape of the recessed portion **1026** should preferably correspond to the shape of the first end of the storage member **1030.** In other embodiments, the first housing member **1020** may not include a recessed portion **1026.** The second housing member **1022** can include an interior space **1028** sized and configured to receive a second end of the storage member **1030.** In some embodiments, the interior space **1028** can include a housing seal **1029** in contact with the second end of the storage member **1030.** In some embodiments, the housing seal **1029** creates a sufficient seal such that little to no gas leaks rearward through the interior space **1028.** In some embodiments, the interior space **1028** can also provide a generally snug fit around the storage member **1030** to ensure that the storage member **1030** generally only translates in a frontward and rearward direction. This advantageously reduces the likelihood of the seal between the second end of the storage member **1030** and the housing seal **1029** from being broken.

With continued reference to **FIG. 28****,** the storage member **1030,** such as the illustrated canister or microcylinder, can include a body portion **1040** and a head **1042.** As shown in the illustrated embodiment, the body portion **1040** can have a generally cylindrical shape with a semi-spherical first end. The body portion **1040,** in conjunction with the head **1042,** can form an internal volume **1041** to contain a fluid such as a gas in cither gaseous or liquid form, or a combination of both, at a first pressure and concentration which can be different than atmospheric gas. For example, such gases can include, but are not limited to, expansile gases, ophthalmic gases such as SF₆, C₃F₈, C₂F₆, or similar gases, propellant gases such as CO₂, refrigerant gases such as N₂0, and other various types of gases. The size of the interior space **1041** can be chosen such that a unit or single-use dose can be contained within the volume. Other shapes can be chosen for the body portion **1040.**

The head **1042** can have a generally tubular shape with an outer diameter matching the inner diameter of the body portion **1040.** The head **1042** can have an internal channel and a flange **1044.** As shown in the illustrated embodiment, the first end of the head **1042** can have an opening with a diameter that matches the diameter of the channel and the second end of the head can have an opening **1046** with a diameter that is less than the diameter of the channel. In some embodiments, the body portion **1040** and the head **1042** can be separate components which are later attached. This potentially advantageously allows for the assembly of internal components of the head **1042** prior to assembly. Once all components are assembled within the head **1042,** the head **1042** can be received within the body portion **1040** and fastened using devices and mechanisms such as adhesives, welding, or the like. In some embodiments, such as that illustrated in **FIG. 28****,** the flange **1044** can abut the body portion **1040** and adhered or welded along this surface. In other embodiments, the body portion **1040** and head **1042** can form an integral unit.

The head **1042** can contain a storage member pressure regulation system, which can form part of a first pressure regulation system, and which can take the form of an internal valve mechanism within the channel. The internal valve mechanism can include a retaining ring **1048,** a valve seat **1050,** an internal biasing member or mechanism **1052** such as a spring, a valve piston **1054,** and a piston seal **1056.** The retaining ring **1048** can be placed within an annular slot **1058** located on the head **1042.** The retaining ring **1048** can be made of an elastic material such that the retaining ring can be deformed prior to fitting into slot **1058.** The valve seat **1050** can be placed between the retaining ring **1048** and the second end of the head **1042.** In some embodiments, the valve seat **1050** can be a ring having an outer diameter approximately equal to the internal diameter of the head **1042.**

The valve piston **1054** can have a generally cylindrical shape and be placed between the seat **1050** and the second end of the head **1042.** The outer diameter of the valve piston **1054** can be chosen to be approximately equal to the internal diameter of the head **1042.** As shown in the illustrated embodiment, the valve piston can include an annular slot configured to receive the piston seal **1056,** fluid pathways **1055** or channels located along the perimeter of the piston, and a protrusion **1057.** The fluid pathways **1055** can be configured to allow fluid to pass between the valve piston **1054** and the head **1042.** In the illustrated embodiment, a total of four fluid pathways are included; however, fewer or greater numbers of pathways can be used. In some embodiments, the protrusion **1057** can be a cylindrical member having a smaller diameter that corresponds to the diameter of the opening **1046.** The protrusion **1057** can be configured to fit within the opening **1046.** In some embodiments, the protrusion **1057** can be flush with the end surface of the head **1042.** In other embodiments, the protrusion **1057** can be recessed within the opening or extend beyond the end surface. A biasing mechanism **1052** can be placed between the seat **1050** and the piston **1054** to apply a force on the valve piston **1054** in a frontward direction such that a seal is formed between the piston seal **1056** and the head **1042.** In other embodiments, other types of valve designs can be used such as a ball valve, poppet valve, or any other valve mentioned herein or known in the art.

In some embodiments, the internal biasing mechanism **1052** can be configured such that, when an activation switch is in a first or "pre-activation" position, the internal valve mechanism will not open as a result of any forces applied to it such as the force applied to the storage member **1030** via the first housing member **1020** as a result of the rod biasing mechanism **924.** In some embodiments, the internal biasing mechanism **1052** can be configured such that, when an activation switch is in a second or "open" position, the internal valve mechanism will open as a result of forces applied to it. In some embodiments, the internal biasing mechanism **1052** can be configured such that, when an activation switch is in a third or "closed" position, the internal valve mechanism will not open as a result of any forces applied to it such as the force applied to the storage member **1030** via the first housing member **1020** as a result of the rod biasing mechanism **924.**

In some embodiments, the storage member **1030** can include other structures such as filters integrated in portions of the storage member **1030** such as the head **1042.** The storage member **1030** can include membranes or other sealing structures placed over the head **1042** and over the opening **1046** to provide an additional seal which can advantageously extend the shelf life of the storage member **1030.** The membrane or sealing structure can be punctured by a protruding member, such as a pin **1059,** or any other similar release mechanism. In some embodiments, the release mechanism can be a porous material, for example, known as "frit". The storage member **1030** can also include additional valve members which can serve as a relief valve to reduce the likelihood of rupturing if the pressure contained within the storage member **1030** exceeds certain operational limits. The storage member **1030** can also be configured to rupture in a controlled manner to reduce the likelihood of catastrophic failure.

In some embodiments, the storage member **1030,** and the internal components such as the internal valve, is manufactured from materials that are both small and light-weight. The material can also be flexible. In some embodiments, the materials and dimensions of the storage member **1030** can be chosen such that the storage member **1030** resists diffusion of gas through the walls of the storage member **1030.** This can provide the advantage of increasing storage life of the storage member **1030** when a gas is contained therein. In some embodiments, the length of the storage member **1030** from a rearward most end of the body **1040** and a frontward most end of the head **1042** can range from approximately 15mm to approximately 65mm, from approximately 20mm to approximately 45mm, and from approximately 25mm to approximately 35mm, such as 29mm. In some embodiments, the outer diameter of the body **1040** can range from approximately 4mm to approximately 25mm, from approximately 6mm to approximately 20mm, and from approximately 8mm to approximately 15mm, such as 9.5mm. In some embodiments, the outer diameter of the head **1042,** not including a flange portion can range from approximately 2mm to approximately 20mm, from approximately 4mm to approximately 15mm, and from approximately 6mm to approximately 10mm, such as 7.5mm.

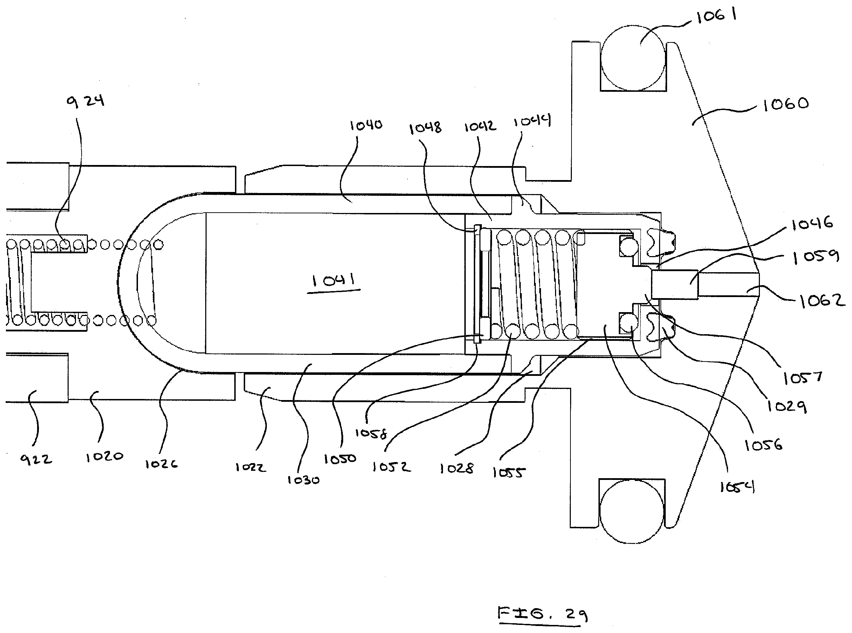

With continued reference to **FIG. 28****,** the second housing member **1022** can include a release mechanism **1059** located within a channel **1062.** The release mechanism **1059** can be substantially centered over the protrusion **1057** of the valve piston **1054** and have a diameter which matches the diameter of the opening **1046.** As illustrated in **FIG. 29****,** during operation, when the storage member **1030** is translated in a frontward direction towards the release mechanism **1059,** the release mechanism **1059** remains stationary such that the release mechanism **1059** can cause the valve piston **1056** to unseat from the head **1042** thereby allowing the flow of fluid from the storage member **1030,** past the pathways **1055** and the release mechanism **1059,** and through the channel **1062** where it ultimately can flow into a chamber for the injectable volume, such as a mixing chamber. In some embodiments, the release mechanism **1059** can be made out of a porous material such that the release mechanism **1059** itself serves as a preliminary filtering mechanism for fluid flowing through channel **1062.** In some embodiments, filters can be added between the release mechanism **1059** and the end of the channel **1062** or at any other location to filter out materials.

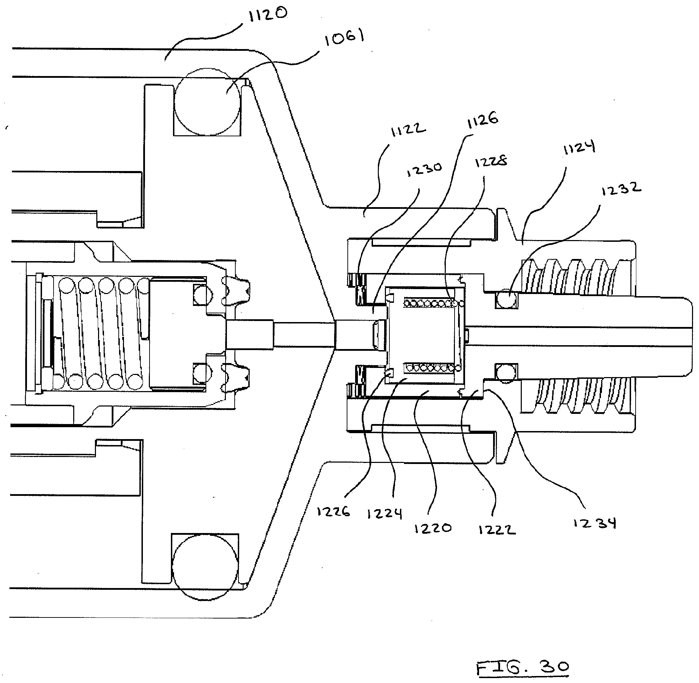

With reference to **FIG. 30****,** an embodiment of a chamber for an injectable volume, such as a mixing chamber, is shown which can include a syringe body **1120,** a syringe pressure regulation system, which can form part of a second pressure regulation system, and various components of the above-mentioned systems. Syringe body **1120** can have a cylindrical body and a nose **1122** at a front end. In some embodiments, a threaded nozzle **1124,** which can include multiple components of a pressure regulation system, can be removably attached to the nose **1122** of the syringe body **1120.** This can advantageously facilitate assembly of the apparatus by allowing the pressure regulation system to be assembled within the smaller nozzle **1124** prior to being incorporated with the syringe body **1120.** The nozzle **1124** can be attached to the nose **1122** using multiple fastening devices and means such as screws, adhesives, snap fits, welding, or the like. The chamber for an injectable volume can be defined by the inner walls of the syringe body **1120** and the plunger seal **1061.** Furthermore, as with other embodiments of the syringe, the syringe body **1120** can also include indicators along its outer surface corresponding to a chosen concentration and a flange at a rear end of the body **1120** configured to be attached to a metering dial.

With continued reference to **FIG. 30****,** an embodiment of the syringe pressure regulation system is shown comprising a valve body **1220,** a valve end **1222,** a valve piston **1224,** a piston seal **1226,** a piston biasing member or mechanism **1228,** a valve biasing member or mechanism **1230,** and a valve end seal **1232.** Similar to other embodiments of the pressure regulation system, the valve body **1220** and valve end **1222** can slidingly translate within the threaded nozzle **1124.**

In a first position, such as that illustrated in **FIG. 30****,** the valve end **1222** can rest against a lip **1234** of the threaded nozzle **1124** due to force exerted by the valve biasing member **1230** on the valve body **1220** and valve end **1222** in a frontward direction. In the first position, the valve piston **1224** and valve seal **1226** can form a seal and limit, or prevent, the passage of fluid through the valve body **1220.** However, when the pressure in the chamber for an injectable volume increases beyond a threshold value to overcome the biasing force exerted by the piston biasing member **1228,** the valve piston **1224** can be translated in a frontward direction against the force applied by the piston biasing member **1228** and fluid can pass through the valve body **1220** and valve end **1222** and into the atmosphere. Once the pressure reduces back to a threshold value, the equilibrium of forces allows the valve piston **1224** and valve seal **1226** to once again sealingly contact the valve body **1220.**

In a second position, the valve body **1220** and valve end **1222** can be translated in a rearward direction against the valve biasing member **1230.** For example, this can be accomplished by applying a force in the rearward direction upon the valve end **1222.** In the second position, contact between the valve piston **1224** and an internal protruding member **1126** of the syringe body **1120** can cause the valve piston **1224** to move in a rearward direction relative to the valve body **1220** and valve end **1222** such that the valve piston **1224** no longer sealingly contacts the valve body **1220.** This could, in some embodiments, allow the passage of fluid to and from the chamber for an injectable volume. In some embodiments, the pressure regulation system can be forced into a second position when an inline filter is threaded onto the threaded nozzle **1124.** For example, an attachment **760** as illustrated in **FIG. 14****.** Other types of attachments, such as stopcocks, valves, tubing, or the like, can also be attached to the threaded nozzle **1124.**

### External Gas Filling

In some embodiments, the pressurized chamber can be external to the apparatus. In such embodiments, the pressurized chamber can be a tank or other canister containing the gas in liquid or gaseous (or a combination) form. In some embodiments, the tank can be attached to the threaded nozzle via tubing or other mechanisms. The connection between the threaded nozzle and tubing can cause the pressure regulation system located on the apparatus to be forced open thereby allowing the gas from the tank to be input into the chamber. In some embodiments, introduction of the gas from the tank can be performed during a first phase of operation. As such, the gas from the tank can fill the apparatus with gas until the apparatus reaches a configured first volume. In some embodiments, the tank can have a regulator such that the apparatus is filled with gas at a regulated pressure. The connection can then be removed from the threaded nozzle, allowing the valve to function normally. In some embodiments, since the gas can be at a higher pressure than atmospheric air and can exceed a threshold value for the pressure regulation system, the gas can be expelled or bled from the system until a configured pressure is achieved in the apparatus. Once the configured pressure is achieved in the apparatus, the remaining phases of operation can then be completed in a similar manner to those in the above-described embodiments.

The foregoing description is that of an apparatus for mixing and/or injecting gases having certain features, aspects, and advantages in accordance with the present inventions. Various changes and modifications also can be made to the above-described gas mixture apparatus without departing from the scope of the invention. Thus, for example, those skilled in the art will recognize that the invention can be embodied or carried out in a manner that achieves or optimizes one advantage or group of advantages as taught herein without necessarily achieving other objects or advantages as can be taught or suggested herein. In addition, while a number of variations of the invention have been shown and described in detail, other modifications and methods of use, which are within the scope of this invention, will be readily apparent to those of skill in the art based upon this disclosure. It is contemplated that various combinations or subcombinations of the specific features and aspects of the embodiments can be made and still fall within the scope of the invention. Accordingly, it should be understood that various features and aspects of the disclosed embodiments can be combined with or substituted for one another in order to form varying modes of the disclosed gas mixture apparatus.

## Claims

1. A hand-held gas mixer (10a, 10b) and injector assembly for mixing gases comprising a gas and atmospheric air, comprising:
a syringe body (520, 1120) comprising a cylindrical body, an aperture (522) at a rear end and an outlet at a front end, the outlet comprising a threaded nozzle (524, 1124) with threads on the interior and a pressure regulation system (710a, 710b) disposed in the nozzle (524, 1124), the pressure regulation system (710a, 710b) comprising a valve (720, 1220) having a closed configuration and an open configuration, wherein the pressure regulation system (710a, 71ab) is configured to serve as a separation mechanism between both the mixing chamber (510a) and the surrounding atmosphere;
a plunger slidably disposed in the syringe body and, with the syringe body, defining a mixing chamber (510a, 510b) within the syringe body configured to receive air from the atmosphere to mix with the gas, received from a pressure chamber (310a) and/or already existing within the mixing chamber (510a, 510b), the mixing chamber fluidically connected via the pressure regulation system (710a, 710b) with the atmosphere;
wherein the pressure regulation system (710a, 710b) further comprises an attachment comprising a filter (760) having a first open end (762) with a flange configured to engage with the nozzle (525, 1124), and a second open end (766), and a filter element (768) located therebetween, the attachment configured such that as it is threaded to the nozzle (524, 1124) it engages with and opens the valve to the open configuration to allow atmospheric air to flow through the attachment (760) and the open valve (720, 1220) into the mixing chamber (510b) for mixing with the gas in the mixing chamber (510b).

2. The hand-held gas mixer and injector assembly of Claim 1, wherein the attachment (760) is further configured to engage the valve (720, 1220) and translate the valve against a biasing force of a biasing mechanism (730) towards the rear end of the syringe body (520).

3. The hand-held gas mixer and injector assembly of Claim 1, wherein the valve comprises a valve body (1220), a valve end (1222), a piston (1224), a seal (1226), a piston biasing member (1228), a valve biasing member (1230), a valve end seal (1232), and an internal protruding member (1126).

4. The hand-held gas mixer and injector assembly of Claim 3, wherein at least a portion of an inner surface of the attachment (760) tapers inwards, from the first end (726) towards the second end (766) of the attachment (760), so as to correspond to a shape of the valve body (1220).

5. The hand-held gas mixer and injector assembly of Claim 1, further comprising a filling mechanism configured to direct a first fluid into the first chamber so as to move the plunger and expand the first chamber.

6. The hand-held gas mixer and injector assembly of Claim 5, further comprising a second chamber disposed within at least one of the syringe body and the plunger, the second chamber comprising a seal and an internal volume containing at least the first fluid in a concentration different than that in the atmospheric air and at a pressure greater than that of the surrounding atmospheric air.

7. The hand-held gas mixer and injector assembly of Claim 6, wherein the second chamber is a storage member, the second chamber comprises an opening at a first end and an internal valve mechanism located adjacent the first end, the internal valve mechanism configured to seal the opening.

8. The hand-held gas mixer and injector assembly of Claim 7, wherein the internal valve mechanism comprises a piston, a seal, and a biasing member, the internal valve mechanism configured to seal the opening at least prior to activation of the assembly.

9. The hand-held gas mixer and injector assembly of Claim 6, wherein the second chamber comprises a relief valve configured to release the first fluid from the second chamber when the pressure within the second chamber exceeds a preconfigured value.

10. The hand-held gas mixer and injector assembly of Claim 1, further comprising a release mechanism, wherein the release mechanism comprises a pin.

11. The hand-held gas mixer and injector assembly of Claim 1, further comprising an measurement control system (110b) comprising a metering dial (120) wherein the metering dial is configured to limit movement of the plunger, via one or more variable stops of the measurement control system (110b), to a plurality of user-selectable positions corresponding to a plurality of volumes of the first chamber.

12. The hand-held gas mixer and injector assembly of Claim 1, wherein the plunger is slidably disposed within the syringe body and moveable along an axial direction, the outlet comprising an outlet passage extending along the axial direction.

## Patentansprüche

1. Handgeführte Gasmischer- (10a, 10b) und Injektoranordnung zum Mischen von Gasen, die ein Gas und atmosphärische Luft umfassen, umfassend:
einen Spritzenkörper (520, 1120), der einen zylindrischen Körper, eine Öffnung (522) an einem hinteren Ende und einen Auslass an einem vorderen Ende umfasst, wobei der Auslass eine Gewindedüse (524, 1124) mit Gewinde im Inneren und ein in der Düse (524, 1124) angeordnetes Druckregelsystem (710a, 710b) umfasst, wobei das Druckregelsystem (710a, 710b) ein Ventil (720, 1220) mit einer geschlossenen Konfiguration und einer offenen Konfiguration umfasst, wobei das Druckregelsystem (710a, 71ab) dazu ausgelegt ist, als Trennmechanismus zwischen der Mischkammer (510a) sowie der umgebenden Atmosphäre zu dienen;
einen Stößel, der verschiebbar in dem Spritzenkörper angeordnet ist und mit dem Spritzenkörper eine Mischkammer (510a, 510b) innerhalb des Spritzenkörpers definiert, die dazu ausgelegt ist, Luft aus der Atmosphäre aufzunehmen, um diese mit dem Gas zu vermischen, das von einer Druckkammer (310a) empfangen wird und/oder bereits in der Mischkammer (510a, 510b) vorhanden ist, wobei die Mischkammer über das Druckregelsystem (710a, 710b) mit der Atmosphäre fluidisch verbunden ist;
wobei das Druckregelsystem (710a, 710b) ferner einen Aufsatz umfasst, umfassend einen Filter (760), der ein erstes offenes Ende (762) mit einem Flansch aufweist, welcher dazu ausgelegt ist, mit der Düse (525, 1124) in Eingriff zu treten, und ein zweites offenes Ende (766) und ein dazwischen angeordnetes Filterelement (768), wobei der Aufsatz so ausgelegt ist, dass er, wenn er mit der Düse (524, 1124) verschraubt wird, mit dem Ventil in Eingriff tritt und es in die geöffnete Konfiguration bringt, um zu ermöglichen, dass atmosphärische Luft durch den Aufsatz (760) und das offene Ventil (720, 1220) in die Mischkammer (510b) strömt, um sich mit dem Gas in der Mischkammer (510b) zu vermischen.

2. Handgeführte Gasmischer- und Injektoranordnung nach Anspruch 1, wobei der Aufsatz (760) ferner dazu ausgelegt ist, mit dem Ventil (720, 1220) in Eingriff zu treten und das Ventil gegen eine Vorspannkraft eines Vorspannmechanismus (730) in Richtung des hinteren Endes des Spritzenkörpers (520) zu verschieben.

3. Handgeführte Gasmischer- und Injektoranordnung nach Anspruch 1, wobei das Ventil einen Ventilkörper (1220), ein Ventilende (1222), einen Kolben (1224), eine Dichtung (1226), ein Kolbenvorspannelement (1228), ein Ventilvorspannelement (1230), eine Ventilenddichtung (1232) und ein internes vorstehendes Element (1126) umfasst.

4. Handgeführte Gasmischer- und Injektoranordnung nach Anspruch 3, wobei sich wenigstens ein Abschnitt einer Innenfläche des Aufsatzes (760) von dem ersten Ende (726) nach innen zu dem zweiten Ende (766) des Aufsatzes (760) verjüngt, um einer Form des Ventilkörpers (1220) zu entsprechen.

5. Handgeführte Gasmischer- und Injektoranordnung nach Anspruch 1, ferner umfassend einen Füllmechanismus, der dazu ausgelegt ist, ein erstes Fluid in die erste Kammer zu leiten, um den Stößel zu bewegen und die erste Kammer auszuweiten.

6. Handgeführte Gasmischer- und Injektoranordnung nach Anspruch 5, ferner umfassend eine zweite Kammer, die in wenigstens einem von dem Spritzenkörper und dem Stößel angeordnet ist, wobei die zweite Kammer eine Dichtung und ein Innenvolumen umfasst, das wenigstens das erste Fluid in einer Konzentration enthält, die sich von der in der atmosphärischen Luft unterscheidet, und bei einem Druck, der größer als der der umgebenden atmosphärischen Luft ist.

7. Handgeführte Gasmischer- und Injektoranordnung nach Anspruch 6, wobei die zweite Kammer ein Speicherelement ist, die zweite Kammer eine Öffnung an einem ersten Ende und einen internen Ventilmechanismus umfasst, der angrenzend an das erste Ende angeordnet ist, wobei der interne Ventilmechanismus dazu ausgelegt ist, die Öffnung abzudichten.

8. Handgeführte Gasmischer- und Injektoranordnung nach Anspruch 7, wobei der innere Ventilmechanismus einen Kolben, eine Dichtung und ein Vorspannelement umfasst, wobei der innere Ventilmechanismus dazu ausgelegt ist, die Öffnung wenigstens vor Aktivierung der Anordnung abzudichten.

9. Handgeführte Gasmischer- und Injektoranordnung nach Anspruch 6, wobei die zweite Kammer ein Entlastungsventil umfasst, das dazu ausgelegt ist, das erste Fluid aus der zweiten Kammer freizugeben, wenn der Druck in der zweiten Kammer einen vorkonfigurierten Wert überschreitet.

10. Handgeführte Gasmischer- und Injektoranordnung nach Anspruch 1, ferner umfassend einen Freigabemechanismus, wobei der Freigabemechanismus einen Stift umfasst.

11. Handgeführte Gasmischer- und Injektoranordnung nach Anspruch 1, ferner umfassend ein Messsteuersystem (110b), das ein Dosierwählrad (120) umfasst, wobei das Dosierwählrad dazu ausgelegt ist, die Bewegung des Stößels über einen oder mehrere variable Anschläge des Messsteuersystems (110b) auf mehrere vom Benutzer wählbare Positionen zu begrenzen, die mehreren Volumina der ersten Kammer entsprechen.

12. Handgeführte Gasmischer- und Injektoranordnung nach Anspruch 1, wobei der Stößel verschiebbar innerhalb des Spritzenkörpers angeordnet und entlang einer axialen Richtung beweglich ist, wobei der Auslass einen Auslassdurchgang umfasst, der sich entlang der axialen Richtung erstreckt.

## Revendications

1. Ensemble de mélangeur (10a, 10b) et d'injecteur de gaz portatif pour le mélange de gaz comprenant un gaz et de l'air atmosphérique, comprenant :
un corps de seringue (520, 1120) comprenant un corps cylindrique, une ouverture (522) à une extrémité arrière et une sortie à une extrémité avant, la sortie comprenant une buse filetée (524, 1124) avec des filetages à l'intérieur et un système de régulation de pression (710a, 710b) disposé dans la buse (524, 1124), le système de régulation de pression (710a, 710b) comprenant une soupape (720, 1220) ayant une configuration fermée et une configuration ouverte, le système de régulation de pression (710a, 71ab) étant configuré pour servir de mécanisme de séparation entre à la fois la chambre de mélange (510a) et l'atmosphère environnante ;
un piston disposé coulissant dans le corps de seringue et définissant, avec le corps de seringue, une chambre de mélange (510a, 510b) à l'intérieur du corps de seringue configurée pour recevoir de l'air provenant de l'atmosphère pour se mélanger avec le gaz, reçu d'une chambre de pression (310a) et/ou existant déjà à l'intérieur de la chambre de mélange (510a, 510b), la chambre de mélange étant fluidiquement reliée à l'atmosphère par l'intermédiaire du système de régulation de pression (710a, 710b) ;
le système de régulation de pression (710a, 710b) comprenant en outre un élément de fixation comprenant un filtre (760) ayant une première extrémité ouverte (762) dotée d'une collerette configurée pour se mettre en prise avec la buse (525, 1124), et une deuxième extrémité ouverte (766), et un élément filtrant (768) placé entre eux, l'élément de fixation étant conçu de telle sorte qu'elle est vissée sur la buse (524, 1124), elle s'engage avec la soupape et l'ouvre en configuration ouverte pour permettre à l'air atmosphérique de s'écouler à travers l'élément de fixation (760) et la soupape ouverte (720, 1220) dans la chambre de mélange (510b) pour se mélanger avec le gaz dans la chambre de mélange (510b).

2. Ensemble de mélangeur et d'injecteur de gaz portatif selon la revendication 1, l'élément de fixation (760) étant en outre configuré pour engager la soupape (720, 1220) et translater la soupape contre une force de sollicitation d'un mécanisme de sollicitation (730) vers l'extrémité arrière du corps de seringue (520).

3. Ensemble de mélangeur et d'injecteur de gaz portatif selon la revendication 1, la soupape comprenant un corps de soupape (1220), une extrémité de soupape (1222), un piston (1224), un joint (1226), un élément de sollicitation de piston (1228), un élément de sollicitation de soupape (1230), un joint d'extrémité de soupape (1232) et un élément saillant interne (1126).

4. Ensemble de mélangeur et d'injecteur de gaz portatif selon la revendication 3, au moins une partie d'une surface intérieure de l'élément de fixation (760) se rétrécissant vers l'intérieur, de la première extrémité (726) vers la seconde extrémité (766) de l'élément de fixation (760), de manière à correspondre à une forme du corps de soupape (1220).

5. Ensemble de mélangeur et d'injecteur de gaz portatif selon la revendication 1, comprenant en outre un mécanisme de remplissage configuré pour diriger un premier fluide dans la première chambre de manière à déplacer le piston et à dilater la première chambre.

6. Ensemble de mélangeur et d'injecteur de gaz portatif selon la revendication 5, comprenant en outre une seconde chambre disposée à l'intérieur d'au moins l'un du corps de seringue et du piston, la seconde chambre comprenant un joint et un volume interne contenant au moins le premier fluide dans une concentration différente de celle dans l'air atmosphérique et à une pression supérieure à celle de l'air atmosphérique environnant.

7. Ensemble de mélangeur et d'injecteur de gaz portatif selon la revendication 6, la seconde chambre étant un élément de stockage, la seconde chambre comprenant une ouverture à une première extrémité et un mécanisme de soupape interne situé adjacent à la première extrémité, le mécanisme de soupape interne étant configuré pour fermer hermétiquement l'ouverture.

8. Ensemble de mélangeur et d'injecteur de gaz portatif selon la revendication 7, le mécanisme de soupape interne comprenant un piston, un joint d'étanchéité et un élément de sollicitation, le mécanisme de soupape interne étant configuré pour fermer hermétiquement l'ouverture au moins avant l'activation de l'ensemble.

9. Ensemble de mélangeur et d'injecteur de gaz portatif selon la revendication 6, la seconde chambre comprenant une soupape de décharge configurée pour libérer le premier fluide de la seconde chambre lorsque la pression à l'intérieur de la seconde chambre dépasse une valeur préconfigurée.

10. Ensemble de mélangeur et d'injecteur de gaz portatif selon la revendication 1, comprenant en outre un mécanisme de libération, le mécanisme de libération comprenant une broche.

11. Ensemble de mélangeur et d'injecteur de gaz portatif selon la revendication 1, comprenant en outre un système de commande de mesure (110b) comprenant un cadran de dosage (120) le cadran de dosage étant configuré pour limiter le déplacement du piston, par l'intermédiaire d'une ou plusieurs butées variables du système de commande de mesure (110b), vers une pluralité de positions sélectionnables par l'utilisateur correspondant à une pluralité de volumes de la première chambre.

12. Ensemble de mélangeur et d'injecteur de gaz portatif selon la revendication 1, le piston étant disposé de manière coulissante à l'intérieur du corps de seringue et mobile le long d'une direction axiale, la sortie comprenant un passage de sortie s'étendant le long de la direction axiale.
